# EUROPEAN PATENT SPECIFICATION

(11) **EP 1 660 660 B1**
(45) Date of publication and mention of the grant of the patent: **24.07.2013**
(21) Application number: 04761674.3
(22) Date of filing: 16.08.2004
(51) Int. Cl.: C12N 15/31, C07K 14/315, C12P 21/02, C12N 15/63, C07K 19/00, A61K 39/09, A61P 31/04, G01N 33/569, A61K 38/16

(54) **Pharmaceutical compositions for use in the treatment of Streptococcus Pyogenes infection**
Pharmazeutische Zusammensetzungen zur Behandlung von Streptococcus Pyogenes-Infektion
Compositions pharmaceutiques pour traiter l'infection par Streptococcus Pyogenes

(30) Priority: 15.08.2003 US 495094 P
(43) Date of publication of application: 31.05.2006
(73) Proprietor: ID Biomedical Corporation of Quebec, Laval, QC H7V 3S8 (CA)
(72) Inventor: RIOUX, Stéphane, Beauport, Quebec G1E 1J3 (CA); MARTIN, Denis, St-Augustin-de-Desmaures, Quebec G3A 1E9 (CA); HAMEL, Josée, Sillery, Quebec G1T 1N6 (CA); BRODEUR, Bernard, R., Sillery, Quebec G1T 1N6 (CA); RHEAULT, Patrick, St-Etienne-de-Lauzon, Quebec G6J 1P9 (CA); DROUIN, Nathalie, St-Etienne-de-Lauzon, Quebec G6J 1E8 (CA)
(74) Representative: Lubienski, Michael John
(86) International application number: PCT/CA2004/001510
(87) International publication number: WO 2005/017093

(56) References cited:
- WO-A2-02/34771
- CA-A1- 2 413 576
- CA-A1- 2 432 525
- CA-A1- 2 438 921
- US-A1- 2004 071 729
- DATABASE GENBANK [Online] BERES, S.B. ET AL: 'Genome sequence of a serotype M3 strain of group A Streptococcus:Phage-encoded toxins, the high-virulence phenotype, and clone emergence', XP003007939 Database accession no. AE014137 & PROC.NAT.ACAD.SCI.USA vol. 99, no. 15, July 2002, pages 10078 - 10083
- DATABASE GENBANK [Online] FERRETTI J.J. ET AL: 'Complete genome sequence of an M1 strain of Streptococcus pyogenes', XP003007940 Database accession no. AE006478 & PROC.NAT.ACAD.SCI.USA vol. 98, no. 8, April 2001, pages 4658 - 4663
- DATABASE GENESEQ [Online] 19 June 2003 WANG L. ET AL: 'Prokaryotic essential gene #31933', XP003007941 Database accession no. ACA50276 & WO 02 77183 A2
- DATABASE GENESEQ [Online] 19 June 2003 WANG L. ET AL: 'Protein encoded by prokaryotic essential gene #31933', XP003007942 Database accession no. ABU46406 & WO 02 77183 A2
- SCHALLA W O; JOHNSON W: "IMMUNOGENICITY OF RIBOSOMAL VACCINES ISOLATED FROM GROUP A TYPE 14 STREPTOCOCCUS-PYOGENES", INFECTION AND IMMUNITY, vol. 11, no. 6, 1975, pages 1195-1202, ISSN: 0019-9567

## Description

### FIELD OF THE INVENTION

The present invention is related to SHB-GAS-102, SHB-GAS-103, and SHB-GAS-104 polypeptides of S. pyogenes (Group A Streptococcus) and corresponding DNA fragments, which may be used to prevent, diagnose and/or treat S. pyogenes infections.

### BACKGROUND OF THE INVENTION

Streptococci are gram (+) bacteria which are differentiated by group specific carbohydrate antigens A through O which are found at the cell surface. S. pyogenes isolates are further distinguished by type-specific M protein antigens. M proteins are important virulence factors which are highly variable both in molecular weights and in sequences. Indeed, more than 100-M protein types have been identified on the basis of antigenic differences.

S. pyogenes is responsible for many diverse infection types, including pharyngitis, erysipelas and impetigo, scarlet fever, and invasive diseases such as bacteremia and necrotizing fasciitis. A resurgence of invasive disease in recent years has been documented in many countries, including those in North America and Europe. Although the organism is sensitive to antibiotics, the high attack rate and rapid onset of sepsis results in high morbidity and mortality.

To develop a vaccine that will protect individuals from S. pyogenes infection, efforts have focused on virulence factors such as the type-specific M proteins. However, the carboxy-terminal portion of M proteins was found to induce cross-reactive antibodies which reacted with human myocardium, tropomyosin, myosin, and vimentin, which might be implicated in autoimmune diseases. Others have used recombinant techniques to produce complex hybrid proteins containing amino-terminal peptides of M proteins from different serotypes. However, a safe vaccine containing all S. pyogenes serotypes will be highly complex to produce and standardize.

In addition to the serotype-specific antigens, other S. pyogenes proteins have generated interest as potential vaccine candidates. The C5a peptidase, which is expressed by at least S. pyogenes 40 serotypes, was shown to be immunogenic in mice, but its capacity to reduce the level of nasopharyngeal colonization was limited. Other investigators have also focused on the streptococcal pyrogenic exotoxins which appear to play an important role in pathogenesis of infection. Immunization with these proteins prevented the deadly symptoms of toxic shock, but did not prevent colonization.

WO 2002/034771 describes nucleic acids and proteins from streptococcus groups A and B.

Therefore there remains an unmet need for S. pyogenes antigens that may be used as vaccine components for the prophylaxis and/or therapy of S. pyogenes infection.

### SUMMARY OF THE INVENTION

The present invention provides a pharmaceutical composition, uses thereof and methods as defined in the claims.

According to one aspect, the present disclosure provides an isolated polynucleotide encoding a polypeptide having at least 70% identity to a second polypeptide comprising SEQ ID No: 2 (Fig. 2), 4 (Fig. 4), 6(Fig. 6), 22(Fig. 8), 24(Fig. 10), 26(Fig. 12), 28 (Fig. 14), 30(Fig. 16), 32 (Fig. 18), 34 (Fig. 20), 36 (Fig. 22), 38 (Fig. 24), 40 (Fig. 26), 42 (Fig. 28), 44 (Fig. 30) or fragments or analogs thereof.

According to one aspect, the present disclosure relates to polypeptides comprising SEQ ID No : 2, 4, 6, 22, 24, 26, 28, 30, 32, 34, 36, 38, 40, 42, 44 or fragments or analogs thereof.

In other aspects, there are disclosed polypeptides encoded by polynucleotides of the disclosure, pharmaceutical compositions, vectors comprising polynucleotides of the disclosure operably linked to an expression control region, as well as host cells transfected with said vectors and processes for producing polypeptides comprising culturing said host cells under conditions suitable for expression.

### BRIEF DESCRIPTION OF THE DRAWINGS

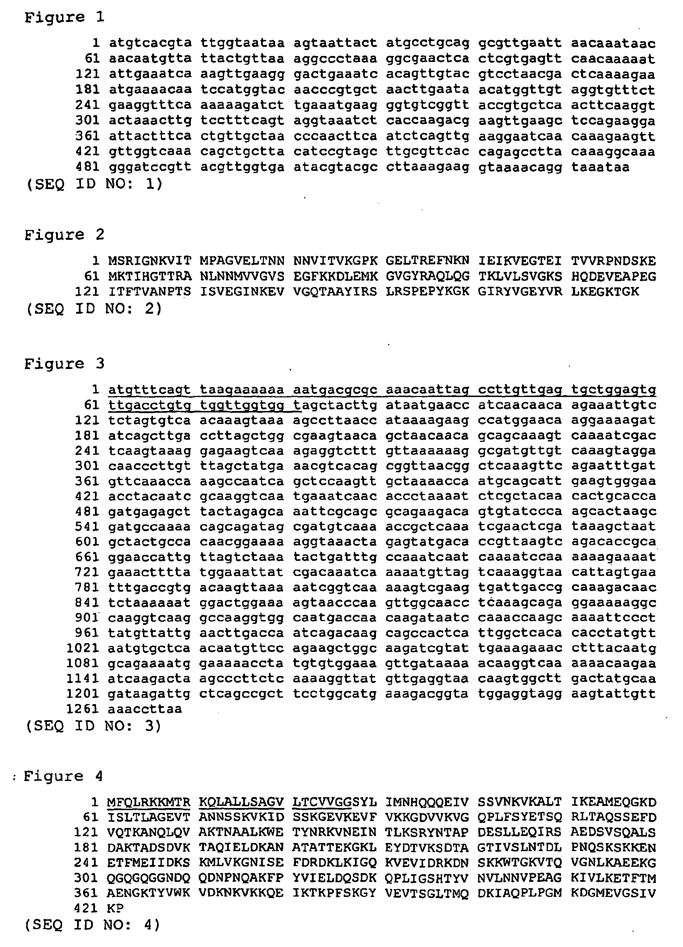

Figure 1 represents the DNA sequence of SHB-GAS-102 gene from serotype M1 S. pyogenes strain ATCC700294; SEQ ID NO: 1.
Figure 2 represents the amino acid sequence SHB-GAS-102 polypeptide from serotype M1 S. pyogenes strain ATCC700294; SEQ ID NO: 2.
Figure 3 represents the DNA sequence of SHB-GAS-103 gene from serotype M1 S. pyogenes strain ATCC700294; SEQ ID NO: 3. The underlined portion of the sequence represents the region coding for the leader peptide.
Figure 4 represents the amino acid sequence SHB-GAS-103 polypeptide from serotype M1 S. pyogenes strain ATCC700294; SEQ ID NO: 4. The underlined sequence represents the 27 amino acid residues leader peptide.

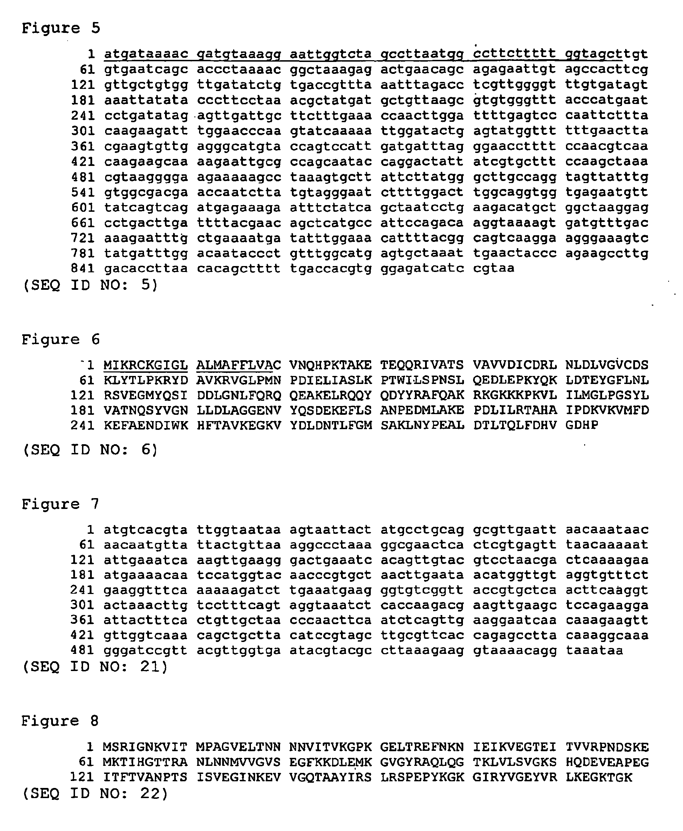

Figure 5 represents the DNA sequence of SHB-GAS-104 gene from serotype M1 S. pyogenes strain ATCC700294; SEQ ID NO: 5. The underlined sequence represents the region coding for the leader peptide.
Figure 6 represents the amino acid sequence SHB-GAS-104 polypeptide from serotype M1 S. pyogenes strain ATCC700294; SEQ ID NO: 6. The underlined sequence represents the 19 amino acid residues leader peptide.
Figure 7 represents the DNA sequence of SHB-GAS-102 gene from serotype M3 S. pyogenes strain MGAS315; SEQ ID NO: 21.
Figure 8 represents the amino acid sequence SHB-GAS-102 protein from M3 S. pyogenes strain MGAS315; SEQ ID NO: 22.

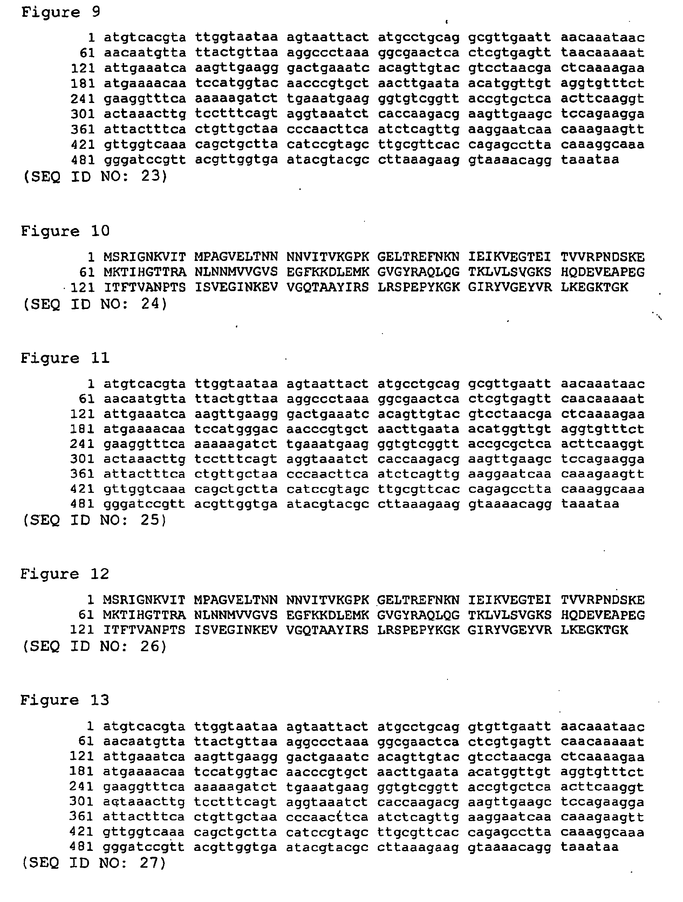

Figure 9 represents the DNA sequence of SHB-GAS-102 gene from serotype M3 S. pyogenes strain SSI-1; SEQ ID NO: 23.
Figure 10 represents the amino acid sequence SHB-GAS-102 protein from M3 S. pyogenes strain SSI-1; SEQ ID NO: 24.
Figure 11 represents the DNA sequence of SHB-GAS-102 gene from serotype M5 S. pyogenes strain Manfredo; SEQ ID NO: 25.
Figure 12 represents the amino acid sequence SHB-GAS-102 protein from M5 S. pyogenes strain Manfredo; SEQ ID NO: 26.
Figure 13 represents the DNA sequence of SHB-GAS-102 gene from serotype M18 S. pyogenes strain MGAS8232; SEQ ID NO: 27.

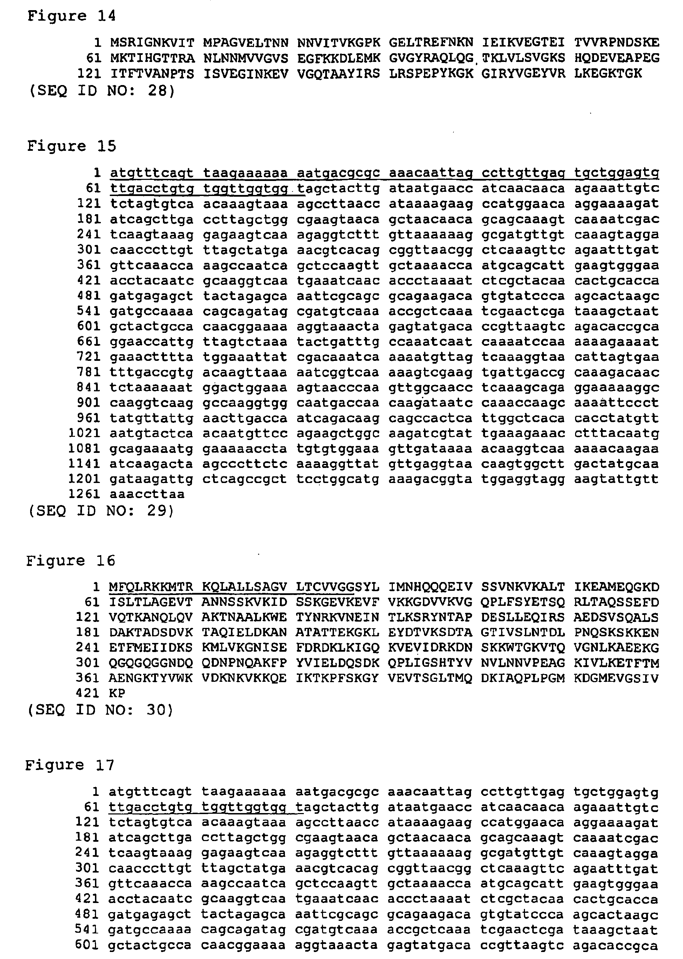

Figure 14 represents the amino acid sequence SHB-GAS-102 protein from M18 S. pyogenes strain MGAS8232; SEQ ID NO: 28.
Figure 15 represents the DNA sequence of SHB-GAS-103 gene from serotype M3 S. pyogenes strain MGAS315; SEQ ID NO: 29. The underlined portion of the sequence represents the region coding for the leader peptide.
Figure 16 represents the amino acid sequence SHB-GAS-103 protein from M3 S. pyogenes strain MGAS315; SEQ ID NO: 30. The underlined sequence represents the 27 amino acid residues leader peptide.

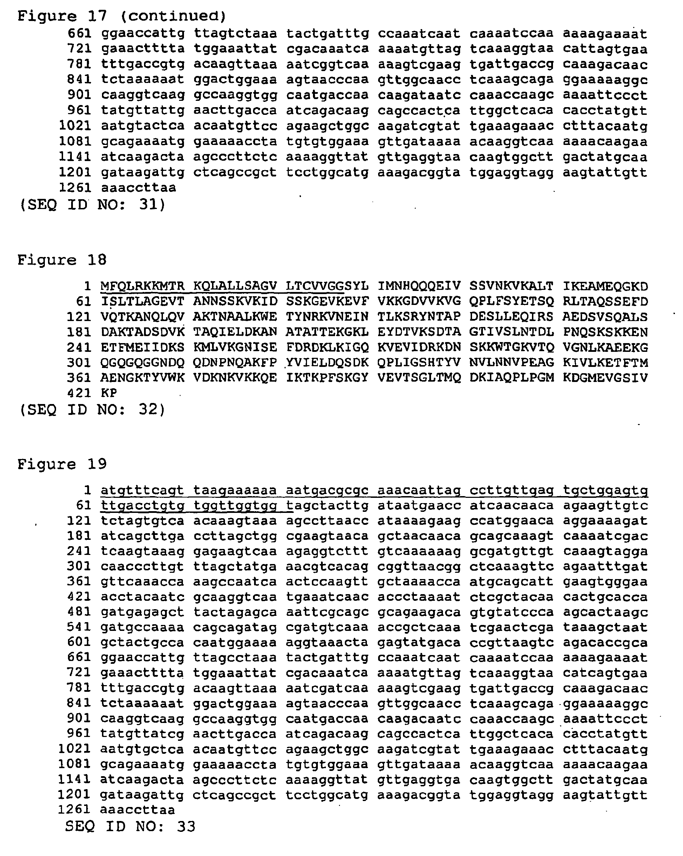

Figure 17 represents the DNA sequence of SHB GAS-103 gene from serotype M3 S. pyogenes strain SSI-1; SEQ ID NO: 31. The underlined portion of the sequence represents the region coding for the leader peptide.
Figure 18 represents the amino acid sequence SHB-GAS-103 protein from M3 S. pyogenes strain SSI-1; SEQ ID NO: 32. The underlined sequence represents the 27 amino acid residues leader peptide.
Figure 19 represents the DNA sequence of SHB-GAS-103 gene from serotype M5 S. pyogenes strain Manfredo; SEQ ID NO: 33. The underlined portion of the sequence represents the region coding for the leader peptide.

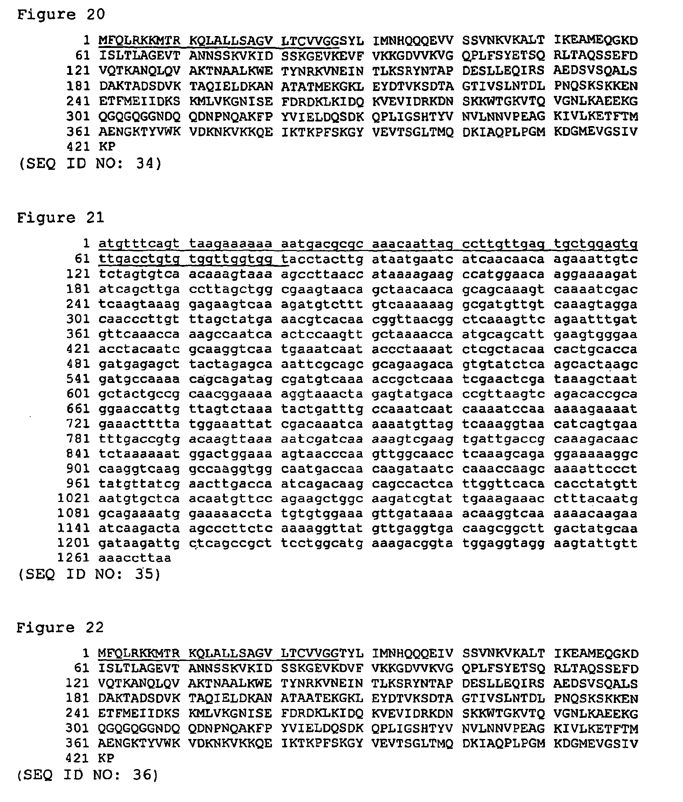

Figure 20 represents the amino acid sequence SHB-GAS-103 protein from M5 S. pyogenes strain Manfredo; SEQ ID NO: 34. The underlined sequence represents the 27 amino acid residues leader peptide.
Figure 21 represents the DNA sequence of SHB-GAS-103 gene from serotype M18 S. pyogenes strain MGAS8232; SEQ ID NO: 35. The underlined portion of the sequence represents the region coding for the leader peptide.
Figure 22 represents the amino acid sequence SHB-GAS-103 protein from M18 S. pyogenes strain MGAS8232; SEQ ID NO: 36. The underlined sequence represents the 27 amino acid residues leader peptide.

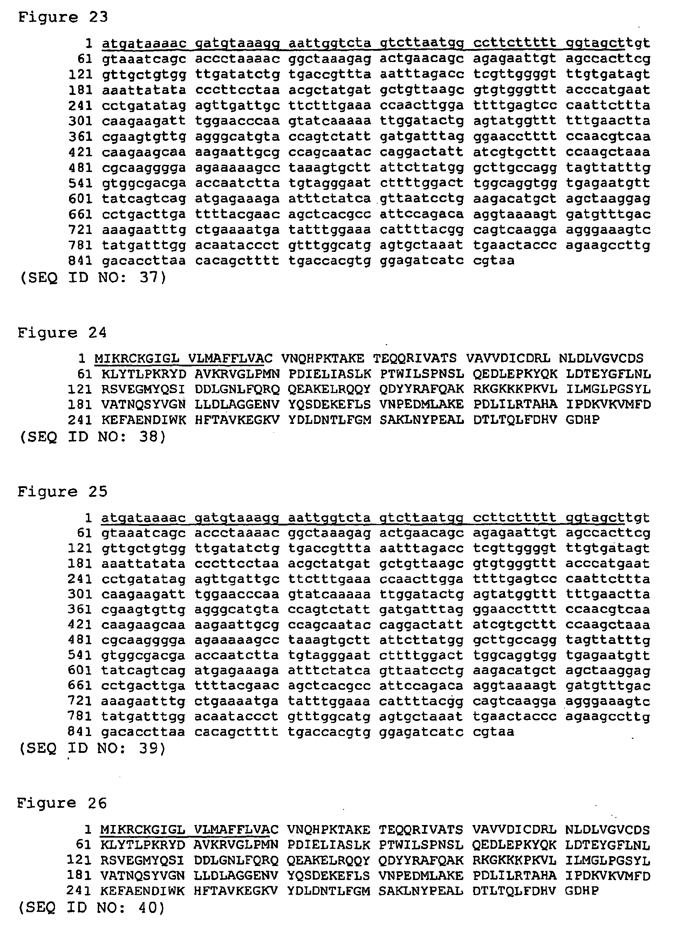

Figure 23 represents the DNA sequence of SHB-GAS-104 gene from serotype M3 S. pyogenes strain MGAS315; SEQ ID NO: 37. The underlined portion of the sequence represents the region coding for the leader peptide.
Figure 24 represents the amino acid sequence SHB-GAS-104 protein from M3 S. pyogenes strain MGAS315; SEQ ID NO: 38. The underlined sequence represents the 19 amino acid residues leader peptide.
Figure 25 represents the DNA sequence of SHB-GAS-104 gene from serotype M3 S. pyogenes strain SSI-1; SEQ ID NO: 39. The underlined portion of the sequence represents the region coding for the leader peptide.
Figure 26 represents the amino acid sequence SHB-GAS-104 protein from M3 S. pyogenes strain SSI-1; SEQ ID NO: 40. The underlined sequence represents the 19 amino acid residues leader peptide.

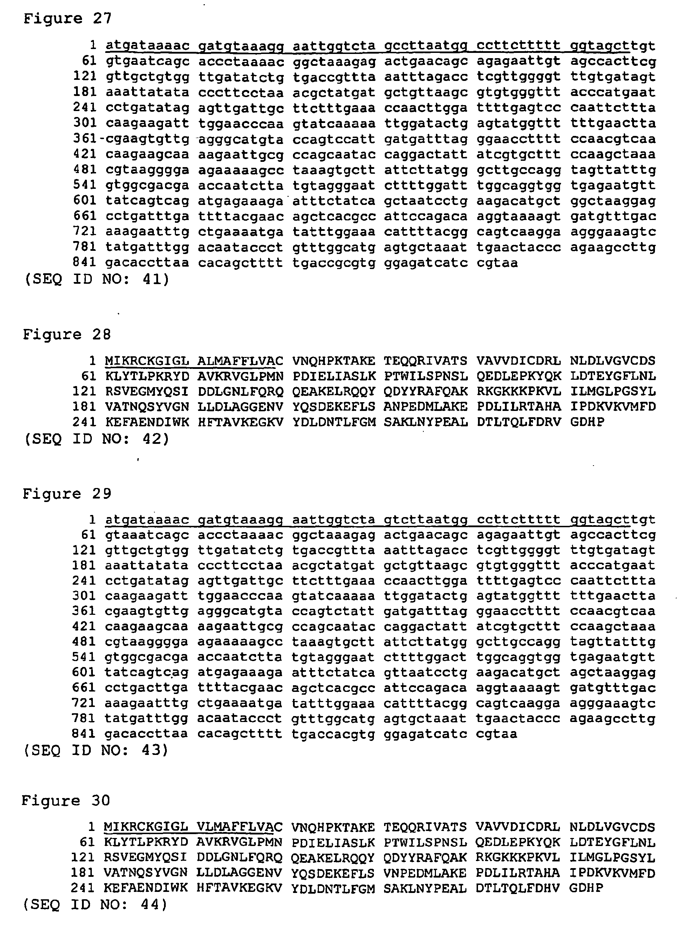

Figure 27 represents the DNA sequence of SHB-GAS-104 gene from serotype M5 S. pyogenes strain Manfredo; SEQ ID NO: 41. The underlined portion of the sequence represents the region coding for the leader peptide.
Figure 28 represents the amino acid sequence SHB-GAS-104 protein from M5 S. pyogenes strain Manfredo; SEQ ID NO: 42. The underlined sequence represents the 19 amino acid residues leader peptide.
Figure 29 represents the DNA sequence of SHB-GAS-104 gene from serotype M18 S. pyogenes strain MGAS8232; SEQ ID NO: 43. The underlined portion of the sequence represents the region coding for the leader peptide.
Figure 30 represents the amino acid sequence SHB-GAS-104 protein from M18 S. pyogenes strain MGAS8232; SEQ ID NO: 44. The underlined sequence represents the 19 amino acid residues leader peptide.

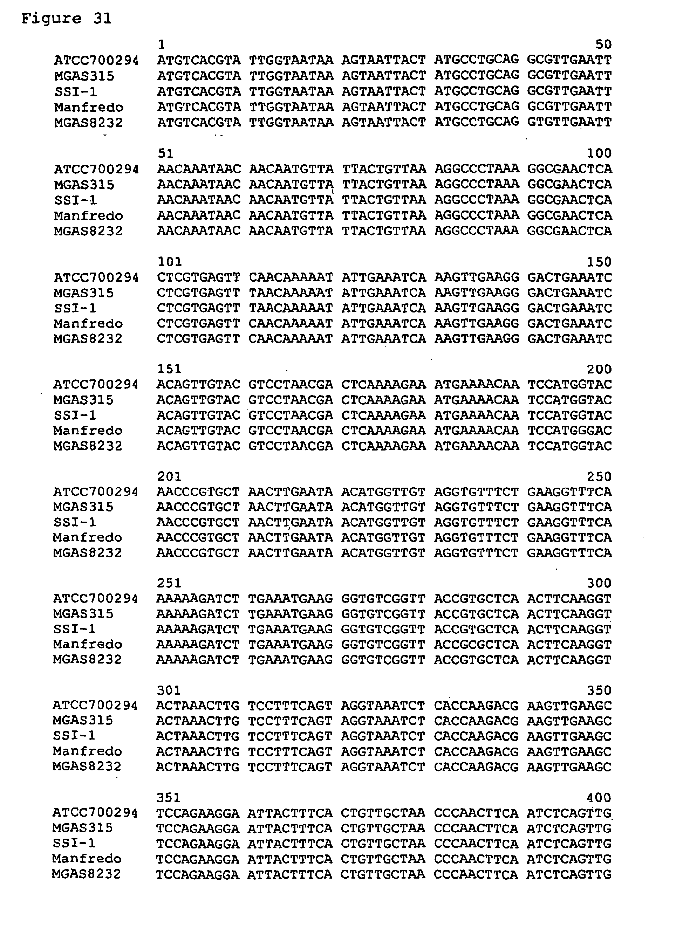

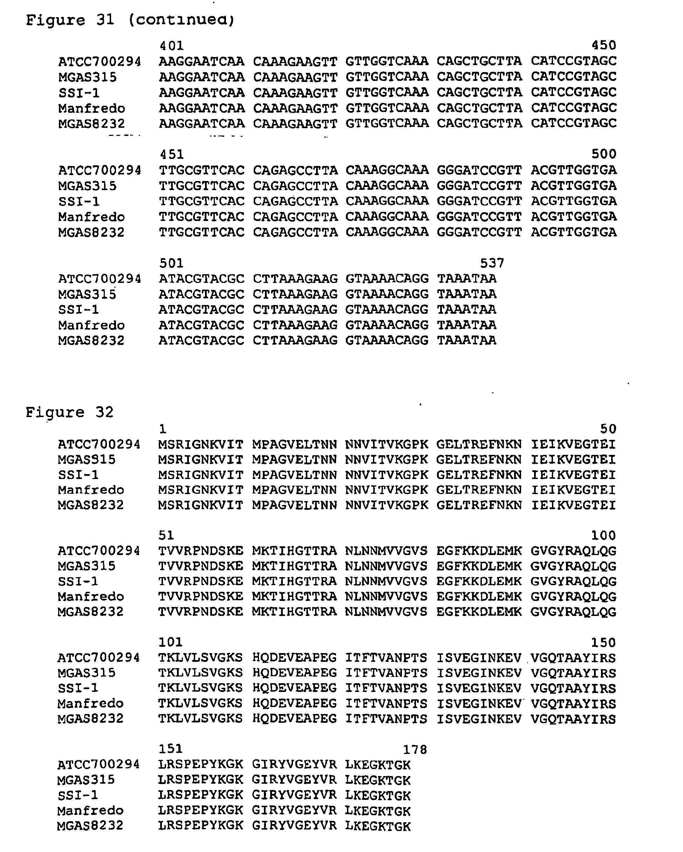

Figure 31 depicts the comparison of the nucleotide sequences of the SHB-GAS-102 genes from the S. pyogenes serotype M1 ATCC700294 (SEQ ID NO:1), serotype M3 MGAS315 (SEQ ID NO:21), serotype M3 SSI-1 (SEQ ID NO:23), serotype M5 Manfredo (SEQ ID NO:25) and serotype M18 MGAS8232 (SEQ ID NO:27) strains by using the program Clustal W from NTI sequence analysis software.
Figure 32 depicts the comparison of the predicted amino acid sequences of the SHB-GAS-102 open reading frames from the S. pyogenes serotype M1 ATCC700294 (SEQ ID NO:2), serotype M3 MGAS315 (SEQ ID NO:22), serotype M3 SSI-1 (SEQ ID NO:24), serotype M5 Manfredo (SEQ ID NO:26) and serotype M18 MGAS8232 (SEQ ID NO:28) strains by using the program Clustal W from NTI sequence analysis software.

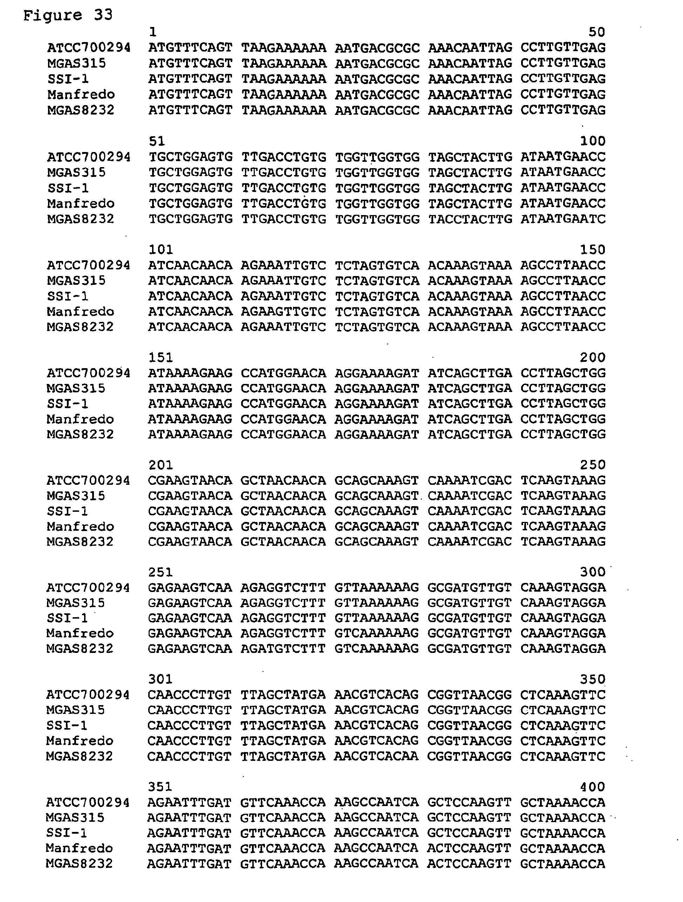

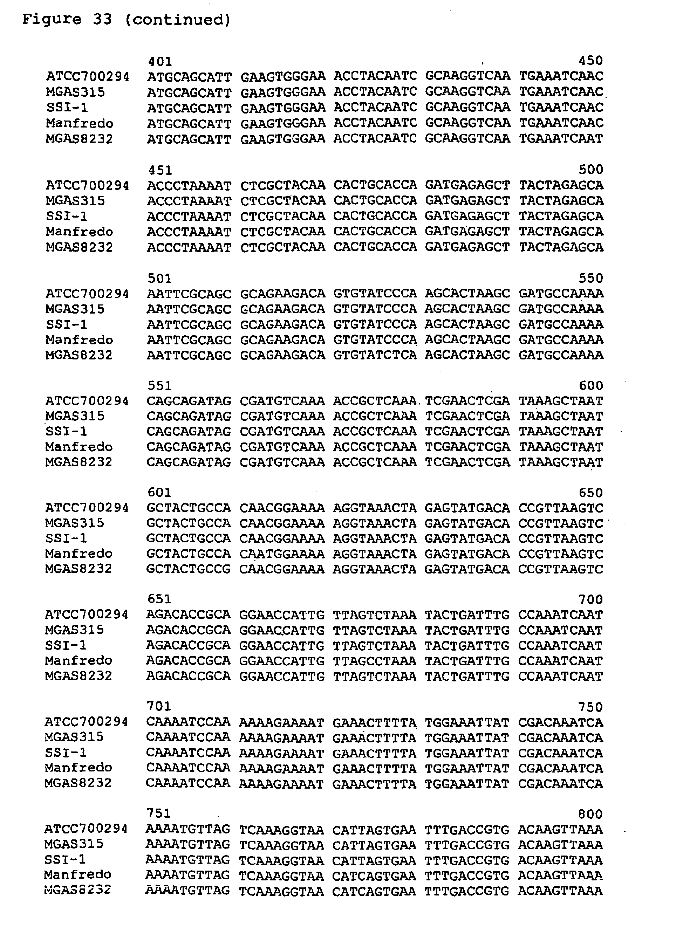

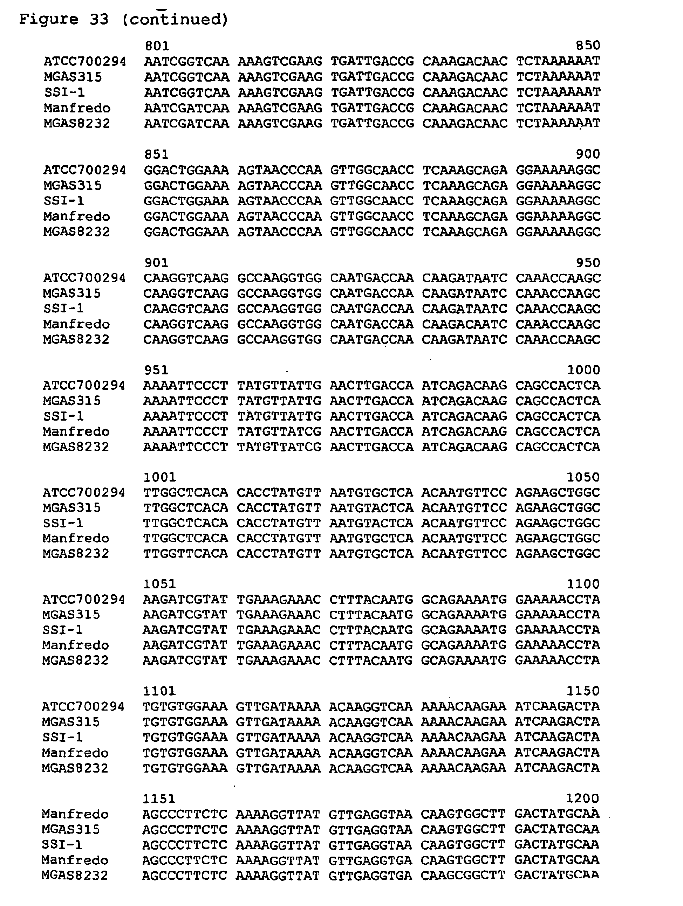

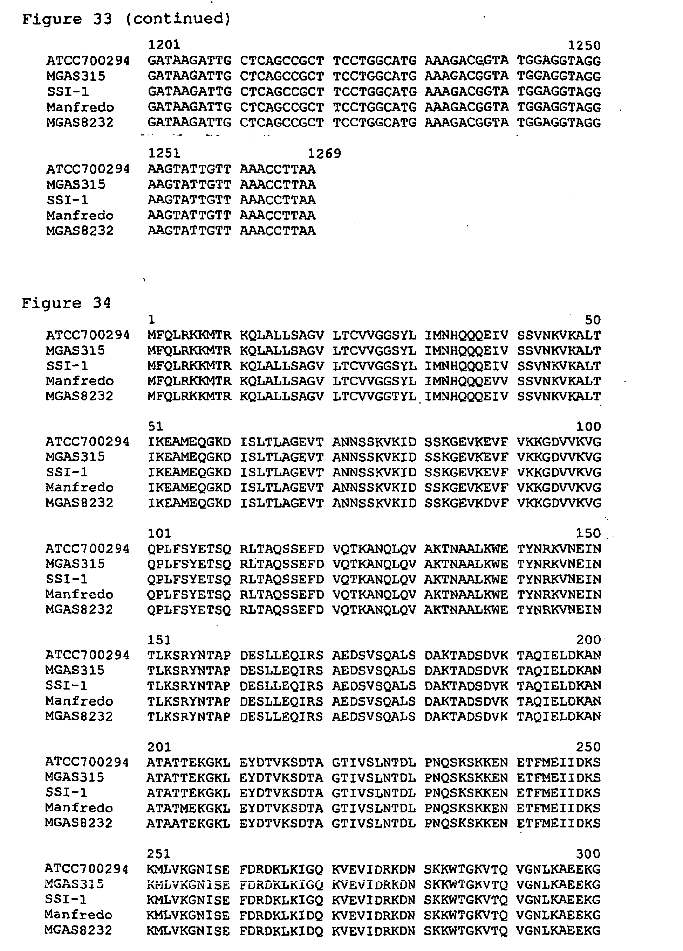

Figure 33 depicts the comparison of the nucleotide sequences of the SHB-GAS-103 genes from the S. pyogenes serotype M1 ATCC700294 (SEQ ID NO:3), serotype M3 MGAS315 (SEQ ID NO:29), serotype M3 SSI-1 (SEQ ID NO:31), serotype M5 Manfredo (SEQ ID NO:33) and serotype M18 MGAS8232, (SEQ ID NO:35) strains by using the program Clustal W from NTI sequence analysis software.

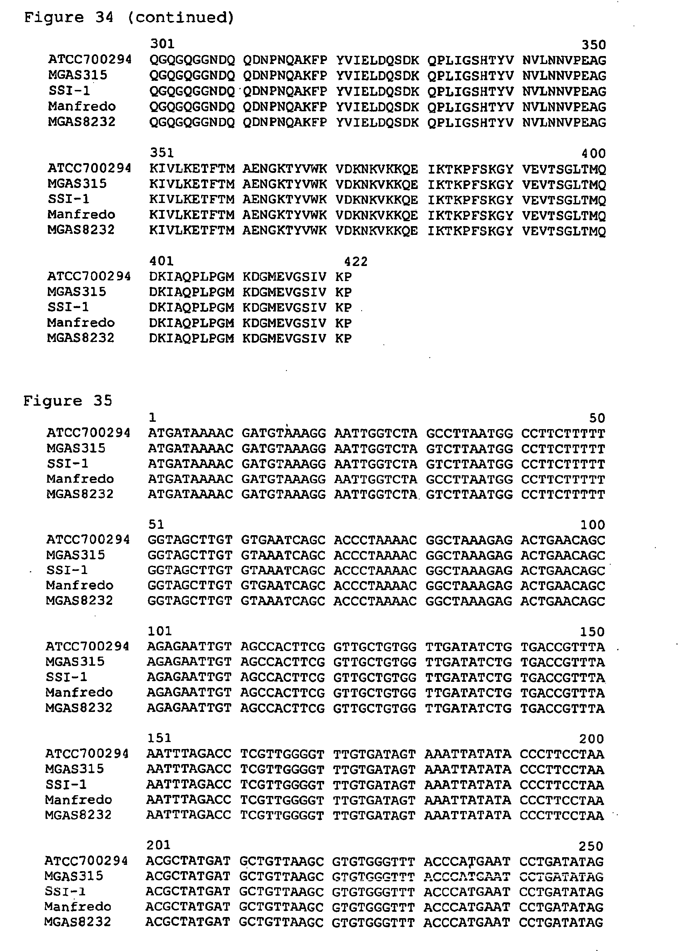

Figure 34 depicts the comparison of the predicted amino acid sequences of the SHB-GAS-103 open reading frames from the S. pyogenes serotype M1 ATCC700294 (SEQ ID NO:4), serotype M3 MGAS315 (SEQ ID NO:30), serotype M3 SSI-1 (SEQ ID NO:32), serotype M5 Manfredo (SEQ ID NO:34) and serotype M18 MGAS8232 (SEQ ID NO:36) strains by using the program Clustal W from NTI sequence analysis software.

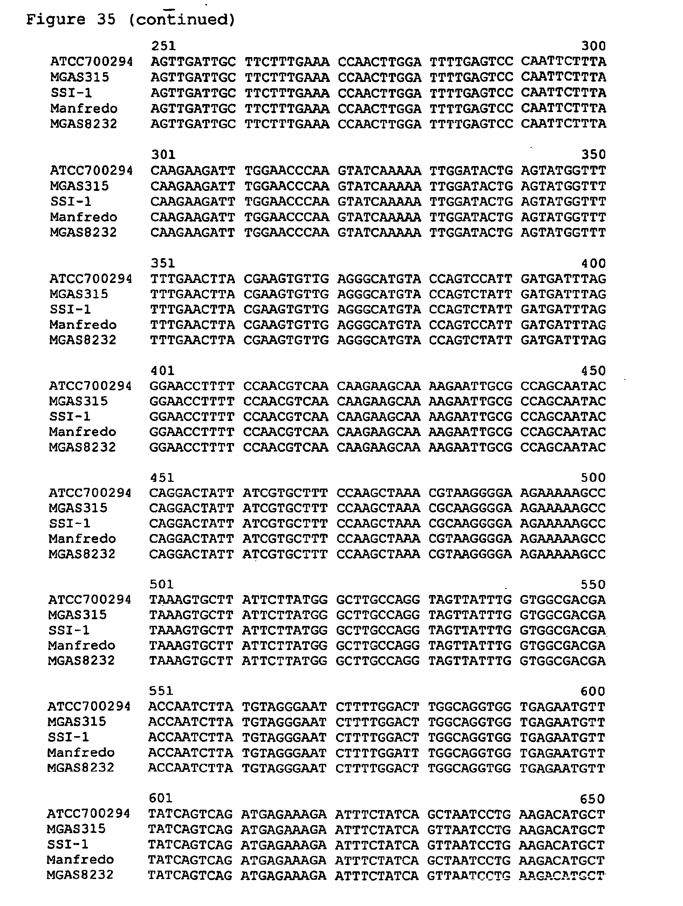

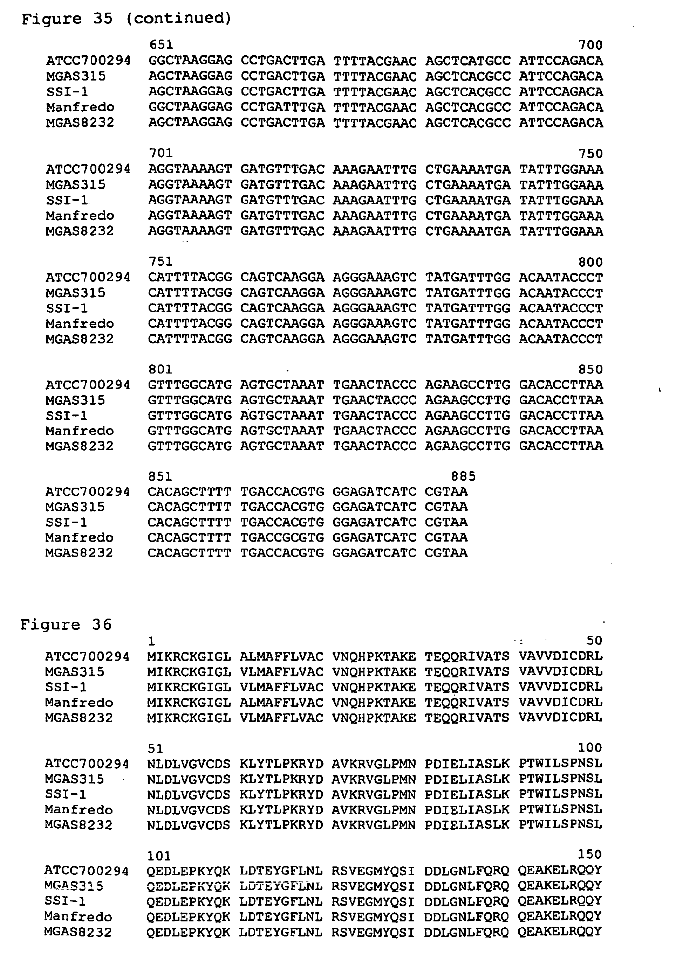

Figure 35 depicts the comparison of the nucleotide sequences of the SHB-GAS-104 genes from the S. pyogenes serotype M1 ATCC700294 (SEQ ID NO:5), serotype M3 MGAS315 (SEQ ID NO:37), serotype M3 SSI-1 (SEQ ID NO:39), serotype M5 Manfredo (SEQ ID NO:41), and serotype M18 MGAS8232 (SEQ ID NO:13) strains by using the program Clustal W from NTI sequence analysis software.

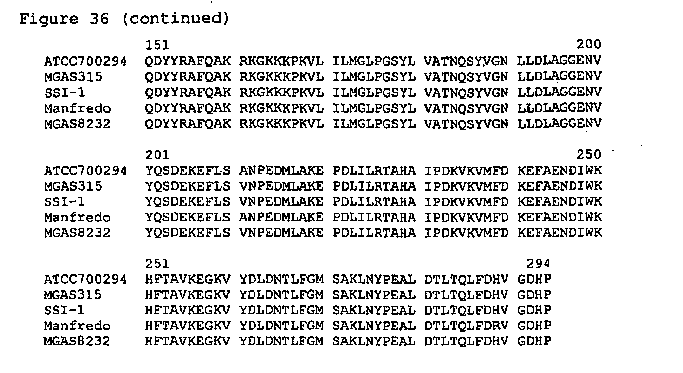

Figure 36 depicts the comparison of the predicted amino acid sequences of the SHB-GAS-104 open reading frames from the S. pyogenes serotype M1 ATCC700294 (SEQ ID NO:6), serotype M3 MGAS315 (SEQ ID NO:38), serotype M3 SSI-1 (SEQ ID NO:40), serotype M5 Manfredo (SEQ ID NO:42) and serotype M18 MGAS3232 (SEQ ID NO:44) strains by using the program Clustal W from NTI sequence analysis software.

### DETAILED DESCRIPTION OF THE INVENTION

The present disclosure provides purified and isolated polynucleotides, which encode S. pyogenes polypeptides which may be used to prevent, diagnose and/or treat S. pyogenes infection.

Disclosed herein are: an isolated polynucleotide encoding a polypeptide having at least 70% identity to a second polypeptide comprising SEQ ID No : 2, 4, 6, 22, 24, 26, 28, 30, 32, 34, 36, 38, 40, 42, 44 or fragments or analogs thereof;
an isolated polynucleotide encoding a polypeptide having at least 80% identity to a second polypeptide comprising SEQ ID No : 2, 4, 6, 22, 24, 26, 28, 30, 32, 34, 36, 38, 40, 42, 44 or fragments or analogs thereof;
an isolated polynucleotide encoding a polypeptide having at least 90% identity to a second polypeptide comprising SEQ ID No : 2, 4, 6, 22, 24, 26, 28, 30, 32, 34, 36, 38, 40, 42, 44 or fragments or analogs thereof;
an isolated polynucleotide encoding a polypeptide having at least 95% identity to a second polypeptide comprising SEQ ID No : 2, 4, 6, 22, 24, 26, 28, 30, 32, 34, 36, 38, 40, 42, 44 or fragments or analogs thereof;
an isolated polynucleotide encoding a polypeptide having at least 98% identity to a second polypeptide comprising SEQ ID No : 2, 4, 6, 22, 24, 26, 28, 30, 32, 34, 36, 38, 40, 42, 44 or fragments or analogs thereof; and
an isolated polynucleotide encoding a polypeptide having at least 99% identity to a second polypeptide comprising SEQ ID No : 2, 4, 6, 22, 24, 26, 28, 30, 32, 34, 36, 38, 40, 42, 44 or fragments or analogs thereof.
Also disclosed herein, are: an isolated polynucleotide encoding a polypeptide having at least 70% identity to a second polypeptide comprising SEQ ID No : 2, 4, 6, 22, 24, 26, 28, 30, 32, 34, 36, 38, 40, 42, 44;
an isolated polynucleotide encoding a polypeptide having at least 80% identity to a second polypeptide comprising SEQ ID No : 2, 4, 6, 22, 24, 26, 28, 30, 32, 34, 36, 38, 40, 42, 44;
an isolated polynucleotide encoding a polypeptide having at least 90% identity to a second polypeptide comprising SEQ ID No : 2, 4, 6, 22, 24, 26, 28, 30, 32, 34, 36, 38, 40, 42, 44;
an isolated polynucleotide encoding a polypeptide having at least 95% identity to a second polypeptide comprising SEQ ID No : 2, 4, 6, 22, 24, 26, 28, 30, 32, 34, 36, 38, 40, 42, 44;
an isolated polynucleotide encoding a polypeptide having at least 98% identity to a second polypeptide comprising SEQ ID No : 2, 4, 6, 22, 24, 26, 28, 30, 32, 34, 36, 38, 40, 42, 44; and
an isolated polynucleotide encoding a polypeptide having at least 99% identity to a second polypeptide comprising SEQ ID No : 2, 4, 6, 22, 24, 26, 28, 30, 32, 34, 36, 38, 40, 42, 44.

According to one aspect, the present disclosure relates to polypeptides comprising SEQ ID No : 2, 4, 6, 22, 24, 26, 28, 30, 32, 34, 36, 38, 40, 42, 44 or fragments or analogs thereof.

According to one aspect, the present disclosure relates to polypeptides comprising SEQ ID No : 2, 4, 6, 22, 24, 26, 28, 30, 32, 34, 36, 38, 40, 42, 44.

Also disclosed herein are: a polynucleotide encoding an epitope bearing portion of a polypeptide comprising SEQ ID No : 2, 4, 6, 22, 24, 26, 28, 30, 32, 34, 36, 38, 40, 42, 44 or fragments or analogs thereof;

a polynucleotide encoding an epitope bearing portion of a polypeptide comprising SEQ ID No : 2, 4, 6, 22, 24, 26, 28, 30, 32, 34, 36, 38, 40, 42, 44 epitope bearing portions of a polypeptide comprising SEQ ID No : 2, 4, 6, 22, 24, 26, 28, 30, 32, 34, 36, 38, 40, 42, 44 or fragments or analogs thereof; and
epitope bearing portions of a polypeptide comprising SEQ ID No : 2, 4, 6, 22, 24, 26, 28, 30, 32, 34, 36, 38, 40, 42, 44.

The present disclosure provides an isolated polynucleotide comprising a polynucleotide chosen from:
(a) a polynucleotide encoding a polypeptide having at least 95% identity to SEQ ID No : 2, 4, 6, 22, 24, 26, 28, 30, 32, 34, 36, 38, 40, 42, 44 or fragments or analogs thereof;
(b) a polynucleotide encoding a polypeptide having at least 98% identity to SEQ ID No : 2, 4, 6, 22, 24, 26, 28, 30, 32, 34, 36, 38, 40, 42, 44 or fragments or analogs thereof;
(c) a polynucleotide encoding a polypeptide having at least 99% identity to SEQ ID No : 2, 4, 6, 22, 24, 26, 28, 30, 32, 34, 36, 38, 40, 42, 44 or fragments or analogs thereof;
(d) a polynucleotide encoding a polypeptide comprising SEQ ID No : 2, 4, 6, 22, 24, 26, 28, 30, 32, 34, 36, 38, 40, 42, 44 or fragments or analogs thereof;
(e) a polynucleotide encoding a polypeptide capable of raising antibodies having binding specificity for a polypeptide comprising SEQ ID No : 2, 4, 6, 22, 24, 26, 28, 30, 32, 34, 36, 38, 40, 42, 44 or fragments or analogs thereof;
(f) a polynucleotide encoding an epitope bearing portion of a polypeptide comprising SEQ ID No : 2, 4, 6, 22, 24, 26, 28, 30, 32, 34, 36, 38, 40, 42, 44 or fragments or analogs thereof;
(g) a polynucleotide comprising SEQ ID No : 1, 3, 5, 21, 23, 25, 27, 29, 31, 33, 35, 37, 39, 41, 43 or fragments or analogs thereof;
(h) a polynucleotide that is complementary to a polynucleotide in (a), (b), (c), (d), (e), (f) or (g).

The present disclosure provides an isolated polynucleotide comprising a polynucleotide chosen from:
(a) a polynucleotide encoding a polypeptide having at least 95% identity to SEQ ID No : 2, 4, 6, 22, 24, 26, 28, 30, 32, 34, 36, 38, 40, 42 or 44;
(b) a polynucleotide encoding a polypeptide having at least 98% identity to SEQ ID No : 2, 4, 6, 22, 24, 26, 28, 30, 32, 34, 36, 38, 40, 42 or 44;
(c) a polynucleotide encoding a polypeptide having at least 99% identity to SEQ ID No : 2, 4, 6, 22, 24, 26, 28, 30, 32, 34, 36, 38, 40, 42 or 44;
(d) a polynucleotide encoding a polypeptide comprising SEQ ID No : 2, 4, 6, 22, 24, 26, 28, 30, 32, 34, 36, 38, 40, 42 or 44;
(e) a polynucleotide encoding a polypeptide capable of raising antibodies having binding specificity for a polypeptide comprising SEQ ID No : 2, 4, 6, 22, 24, 26, 28, 30, 32, 34, 36, 38, 40, 42 or 44;
(f) a polynucleotide encoding an epitope bearing portion of a polypeptide comprising SEQ ID No : 2, 4, 6, 22, 24, 26, 28, 30, 32, 34, 36, 38, 40, 42 or 44;
(g) a polynucleotide comprising SEQ ID No : 1, 3, 5, 21, 23, 25, 27, 29, 31, 33, 35, 37, 39, 41, 43;
(h) a polynucleotide that is complementary to a polynucleotide in (a), (b), (c), (d), (e), (f) or (g).

The present disclosure provides an isolated polynucleotide consisting essentially of a polynucleotide chosen from:
a) a polynucleotide encoding a polypeptide having at least 95% identity to SEQ ID No : 2, 4, 6, 22, 24, 26, 28, 30, 32, 34, 36, 38, 40, 42, 44 or fragments or analogs thereof;
b) a polynucleotide encoding a polypeptide having at least 98% identity to SEQ ID No : 2, 4, 6, 22, 24, 26, 28, 30, 32, 34, 36, 38, 40, 42, 44 or fragments or analogs thereof;
c) a polynucleotide encoding a polypeptide having at least 99% identity to SEQ ID No : 2, 4, 6, 22, 24, 26, 28, 30, 32, 34, 36, 38, 40, 42, 44 or fragments or analogs thereof;
d) a polynucleotide encoding a polypeptide having SEQ ID No : 2, 4, 6, 22, 24, 26, 28, 30, 32, 34, 36, 38, 40, 42, 44 or fragments or analogs thereof;
e) a polynucleotide encoding a polypeptide capable of raising antibodies having binding specificity for a polypeptide having SEQ ID No : 2, 4, 6, 22, 24, 26, 28, 30, 32, 34, 36, 38, 40, 42, 44 or fragments or analogs thereof;
f) a polynucleotide encoding an epitope bearing portion of a polypeptide having SEQ ID No : 2, 4, 6, 22, 24, 26, 28, 30, 32, 34, 36, 38, 40, 42, 44 or fragments or analogs thereof;
g) a polynucleotide having SEQ ID No : 1, 3, 5, 21, 23, 25, 27, 29, 31, 33, 35, 37, 39, 41, 43 or fragments or analogs thereof;
h) a polynucleotide that is complementary to a polynucleotide in (a), (b), (c), (d), (e), (f) or (g)
wherein said polynucleotide encodes a polypeptide that is immunogenic.

The present disclosure provides an isolated polynucleotide consisting essentially of a polynucleotide chosen from:
a) a polynucleotide encoding a polypeptide having at least 95% identity to SEQ ID No : 2, 4, 6, 22, 24, 26, 28, 30, 32, 34, 36, 38, 40, 42 or 44;
b) a polynucleotide encoding a polypeptide having at least 98% identity to SEQ ID No : 2, 4, 6, 22, 24, 26, 28, 30, 32, 34, 36, 38, 40, 42 or 44;
c) a polynucleotide encoding a polypeptide having at least 99% identity to SEQ ID No : 2, 4, 6, 22, 24, 26, 28, 30, 32, 34, 36, 38, 40, 42 or 44;
d) a polynucleotide encoding a polypeptide having SEQ ID No : 2, 4, 6, 22, 24, 26, 28, 30, 32, 34, 36, 38, 40, 42 or 44;
e) a polynucleotide encoding a polypeptide capable of raising antibodies having binding specificity for a polypeptide having SEQ ID No : 2, 4, 6, 22, 24, 26, 28, 30, 32, 34, 36, 38, 40, 42 or 44;
f) a polynucleotide encoding an epitope bearing portion of a polypeptide having SEQ ID No : 2, 4, 6, 22, 24, 26, 28, 30, 32, 34, 36, 38, 40, 42 or 44;
g) a polynucleotide having SEQ ID No : 1, 3, 5, 21, 23, 25, 27, 29, 31, 33, 35, 37, 39, 41 or 43 ;
h) a polynucleotide that is complementary to a polynucleotide in (a), (b), (c), (d), (e), (f) or (g)
wherein said polynucleotide encodes a polypeptide that is immunogenic.

According to one aspect the present disclosure provides an isolated polypeptide Comprising a polypeptide chosen from:
(a) a polypeptide having at least 95% identity to SEQ ID No : 2, 4, 6, 22, 24, 26, 28, 30, 32, 34, 36, 38, 40, 42, 44 or fragments or analogs thereof;
(b) a polypeptide having at least 98% identity to SEQ ID No : 2, 4, 6, 22, 24, 26, 28, 30, 32, 34, 36, 38, 40, 42, 44 or fragments or analogs thereof;
(c) a polypeptide having at least 99% identity to SEQ ID No : 2, 4, 6, 22, 24, 26, 28, 30, 32, 34, 36, 38, 40, 42, 44 or fragments or analogs thereof;
(d) a polypeptide comprising SEQ ID No : 2, 4, 6, 22, 24, 26, 28, 30, 32, 34, 36, 38, 40, 42, 44 or fragments or analogs thereof;
(e) a polypeptide capable of raising antibodies having binding specificity for a polypeptide comprising SEQ ID No : 2, 4, 6, 22, 24, 26, 28, 30, 32, 34, 36, 38, 40, 42, 44 or fragments or analogs thereof;
(f) an epitope bearing portion of a polypeptide comprising SEQ ID No : 2, 4, 6, 22, 24, 26, 28, 30, 32, 34, 36, 38, 40, 42, 44 or fragments or analogs thereof;
(g) the polypeptide of (a), (b), (c), (d), (e) or (f) wherein the N-terminal Met residue is deleted;
(h) the polypeptide of (a), (b), (c), (d), (e) or (f) wherein the secretory amino acid sequence is deleted.

According to one aspect the present disclosure provides an isolated polypeptide comprising a polypeptide chosen from:
(a) a polypeptide having at least 95% identity to SEQ ID No : 2, 4, 6, 22, 24, 26, 28, 30, 32, 34, 36, 38, 40, 42 or 44;
(b) a polypeptide having at least 98% identity to SEQ ID No : 2, 4, 6, 22, 24, 26, 28, 30, 32, 34, 36, 38, 40, 42 or 44;
(c) a polypeptide having at least 99% identity to SEQ ID No : 2, 4, 6, 22, 24, 26, 28, 30, 32, 34, 36, 38, 40, 42 or 44;
(d) a polypeptide comprising SEQ ID No : 2, 4, 6, 22, 24, 26, 28, 30, 32, 34, 36, 38, 40, 42 or 44;
(e) a polypeptide capable of raising antibodies having binding specificity for a polypeptide comprising SEQ ID No : 2, 4, 6, 22, 24, 26, 28, 30, 32, 34, 36, 38, 40, 42 or 44;
(f) an epitope bearing portion of a polypeptide comprising SEQ ID No : 2, 4, 6, 22, 24, 26, 28, 30, 32, 34, 36, 38, 40, 42 or 44;
(g) the polypeptide of (a), (b), (c), (d), (e) or (f) wherein the N-terminal Met residue is deleted,
(h) the polypeptide of (a), (b), (c), (d), (e) or (f) wherein the secretory amino acid sequence is deleted.

According to one aspect, the present disclosure provides an isolated polypeptide, consisting essentially of a polypeptide chosen from:
a) a polypeptide having at least 95% identity to an amino acid sequence having SEQ ID NO: 2, 4, 6, 22, 24, 26, 28, 30, 32, 34, 36, 38, 40, 42, 44 or fragments or analogs thereof;
b) a polypeptide having at least 98% identity to an amino acid sequence having SEQ ID NO: 2, 4, 6, 22, 24, 26, 28, 30, 32, 34, 36, 38, 40, 42, 44 or fragments or analogs thereof;
c) a polypeptide having at least 99% identity to an amino acid sequence having SEQ ID NO: 2, 4, 6, 22, 24, 26, 28, 30, 32, 34, 36, 38, 40, 42, 44 or fragments or analogs thereof;
d) a polypeptide having SEQ ID NO: 2, 4, 6, 22, 24, 26, 28, 30, 32, 34, 36, 38, 40, 42, 44 or fragments or analogs thereof;
e) a polypeptide capable of raising antibodies having binding specificity for a polypeptide having SEQ ID NO: 2, 4, 6, 22, 24, 26, 28, 30, 32, 34, 36, 38, 40, 42, 44 or fragments or analogs thereof;
f) an epitope bearing portion of a polypeptide having SEQ ID NO: 2, 4, 6, 22, 24, 26, 28, 30, 32, 34, 36, 38, 40, 42, 44 or fragments or analogs thereof;
g) the polypeptide of (a), (b), (c), (d), (e) or (f) wherein the N-terminal Met residue is deleted;
h) the polypeptide of (a), (b), (c), d), (e), or (f) wherein the secretory amino acid sequence is deleted
wherein said polypeptide is immunogenic.

According to one aspect, the present disclosure provides an isolated polypeptide, comprising a polypeptide chosen from:
a) a polypeptide having at least 95% identity to an amino acid sequence having SEQ ID NO: 2, 4, 6, 22, 24, 26, 28, 30, 32, 34, 36, 38, 40, 42 or 44;
b) a polypeptide having at least 98% identity to an amino acid sequence having SEQ ID NO: 2, 4, 6, 22, 24, 26, 28, 30, 32, 34, 36, 38, 40, 42 or 44;
c) a polypeptide having at least 99% identity to an amino acid sequence having SEQ ID NO: 2, 4, 6, 22, 24, 26, 28, 30, 32, 34, 36, 38, 40, 42 or 44;
d) a polypeptide comprising SEQ ID NO: 2, 4, 6, 22, 24, 26, 28, 30, 32, 34, 36, 38, 40, 42 or 44;
e) a polypeptide capable of raising antibodies having binding specificity for a polypeptide having SEQ ID NO: 2, 4, 6, 22, 24, 26, 28, 30, 32, 34, 36, 38, 40, 42 or 44;
f) an epitope bearing portion of a polypeptide having SEQ ID NO: 2, 4, 6, 22, 24, 26, 28, 30, 32, 34, 36, 38, 40, 42 or 44;
g) the polypeptide of (a), (b), (c), (d), (e) or (f) wherein the N-terminal Met residue is deleted;
h) the polypeptide of (a), (b), (c), (d), (e), or (f) wherein the secretory amino acid sequence is deleted.

Those skilled in the art will appreciate that the disclosure includes DNA molecules, i.e. polynucleotides and their complementary sequences that encode analogs such as mutants, variants, homologues and derivatives of such polypeptides, as described herein in the present patent application. The disclosure also includes RNA molecules corresponding to the DNA molecules. The disclosure includes the corresponding polypeptides and monospecific antibodies that specifically bind to such polypeptides.

In accordance with the present disclosure, all polynucleotides encoding polypeptides of the present disclosure are within the scope of the present disclosure.

In a further instance, the polypeptides in accordance with the present disclosure are antigenic, i.e. are able to bind specifically to components of the immune response, such as antibodies and lymphocytes.

In a further instance, the polypeptides in accordance with the present disclosure are immunogenic or can elicit an immune response in a host.

In a further instance, the present disclosure also relates to polypeptides which are able to raise antibodies having binding specificity to the polypeptides of the present invention as defined above.

An antibody that "has binding specificity" is an antibody that recognizes and binds the selected polypeptide but which does not substantially recognize and bind other molecules in a sample, e.g., a biological sample, which naturally includes the selected peptide. Specific binding can be measured using an ELISA assay in which the selected polypeptide is used as an antigen.

In a further instance, the polypeptides in accordance with the present disclosure can elicit a B cell response an a host.

In a further instance, the polypeptides in accordance with the present disclosure can elicit a T cell response in a host.

In accordance with the present invention, the polypeptides of the disclosure can also be effective when administered to an host to protect against the bacteria. "Protection" in the biological studies is defined by a significant increase in the survival curve, rate or period. Statistical analysis using the Log rank test to compare survival curves, and Fisher exact test to compare survival rates and numbers of days to death, respectively, can be used to calculate P values and determine whether the difference between the two groups is statistically significant. P values greater than 0.05 are regarded as not significant.

In an additional aspect there are provided antigenic/immunogenic fragments of the polypeptides of the disclosure, or of analogs thereof.

The present disclosure also relates to fragments which are specific to, or for SEQ ID NOS. 2, 4, 6, 22, 24, 26, 28, 30, 32, 34, 36, 38, 40, 42 or 44. A specific fragment contains a defined order of amino acids which occurs in a target polypeptide, and which is characteristic of that target polypeptide, but substantially no other non-target polypeptides. Such polypeptide fragments can be of any size which is necessary to confer specificity, e.g., comprising or consisting of at least 6, 8, 10, 12, 15, 20, 25, 30, 35, 40, 45, 50, 55, 60, 65, 70, 75, 80, 85, 90, 95, 100, 105, 110, 115 amino acids, etc.

Specific fragments can also be described as being specific for Streptococcus pyogenes, indicating that it occurs in that bacteria, but not in other organisms, especially not in GBS or other bacteria.

The fragments of the present disclosure should include one or more such epitopic regions or be sufficiently similar to such regions to retain substantially their antigenic/immunogenic properties. Thus, for fragments according to the present invention the degree of identity is perhaps irrelevant, since they may be 100% identical to a particular part of a polypeptide or analog thereof as described herein.

The present invention further provides fragments having a smaller sequence than the ones described in the figures.

The present invention further provides fragments having at least 10 contiguous amino acid residues from the polypeptide sequences of the present invention.

In one embodiment, at least 15 contiguous amino acid residues.
In one embodiment, at least 20 contiguous amino acid residues.
In one embodiment, at least 25 contiguous amino acid residues.
In one embodiment, at least 30 contiguous amino acid residues.
In one embodiment, at least 35 contiguous amino acid residues.
In one embodiment, at least 40 contiguous amino acid residues.
In one embodiment, at least 45 contiguous amino acid residues.
In one embodiment, at least 50 contiguous amino acid residues.
In one embodiment, at least 55 contiguous amino acid residues.
In one embodiment, at least 60 contiguous amino acid residues.
In one embodiment, at least 65 contiguous amino acid residues.
In one embodiment, at least 70 contiguous amino acid residues.
In one embodiment, at least 75 contiguous amino acid residues.
In one embodiment, at least 80 contiguous amino acid residues.
In one embodiment, at least 85 contiguous amino acid residues.
In one embodiment, at least 90 contiguous amino acid residues.
In one embodiment, at least 95 contiguous amino acid residues.
In one embodiment, at least 100 contiguous amino acid residues.
In one embodiment, at least 105 contiguous amino acid residues.
In one embodiment, at least 110 contiguous amino acid residues.
In one embodiment, at least 115 contiguous amino acid residues.
In one embodiment, at least 120 contiguous amino acid residues.
In one embodiment, at least 125 contiguous amino acid residues.
In one embodiment, at least 130 contiguous amino acid residues.
In one embodiment, at least 135 contiguous amino acid residues.
In one embodiment, at least 140 contiguous amino acid residues.
In one embodiment, at least 145 contiguous amino acid residues.
In one embodiment, at least 150 contiguous amino acid residues.
In one embodiment, at least 155 contiguous amino acid residues.
In one embodiment, at least 160 contiguous amino acid residues.
In one embodiment, at least 165 contiguous amino acid residues.
In one embodiment, at least 170 contiguous amino acid residues.
In one embodiment, at least 175 contiguous amino acid residues.
In one embodiment, at least 180 contiguous amino acid residues.
In one embodiment, at least 185 contiguous amino acid residues.
In one embodiment, at least 190 contiguous amino acid residues.
In one embodiment, at least 195 contiguous amino acid residues.
In one embodiment, at least 200 contiguous amino acid residues.
In one embodiment, at least 205 contiguous amino acid residues.
In one embodiment, at least 210 contiguous amino acid residues.
In one embodiment, at least 215 contiguous amino acid residues.
In one embodiment, at least 220 contiguous amino acid residues.
In one embodiment, at least 225 contiguous amino acid residues.
In one embodiment, at least 230 contiguous amino acid residues.
In one embodiment, at least 235 contiguous amino acid residues.
In one embodiment, at least 240 contiguous amino acid residues.
In one embodiment, at least 245 contiguous amino acid residues.
In one embodiment, at least 250 contiguous amino acid residues.
In one embodiment, at least 255 contiguous amino acid residues.
In one embodiment, at least 260 contiguous amino acid residues.
In one embodiment, at least 265 contiguous amino acid residues.
In one embodiment, at least 270 contiguous amino acid residues.
In one embodiment, at least 275 contiguous amino acid residues.
In one embodiment, at least 280 contiguous amino acid residues.
In one embodiment, at least 285 contiguous amino acid residues.
In one embodiment, at least 290 contiguous amino acid residues.
In one embodiment, at least 295 contiguous amino acid residues.
In one embodiment, at least 300 contiguous amino acid residues.
In one embodiment, at least 305 contiguous amino acid residues.
In one embodiment, at least 310 contiguous amino acid residues.
In one embodiment, at least 315 contiguous amino acid residues.
In one embodiment, at least 320 contiguous amino acid residues.
In one embodiment, at least 325 contiguous amino acid residues.
In one embodiment, at least 330 contiguous amino acid residues.
In one embodiment, at least 335 contiguous amino acid residues.
In one embodiment, at least 340 contiguous amino acid residues.
In one embodiment, at least 345 contiguous amino acid residues.
In one embodiment, at least 350 contiguous amino acid residues.
In one embodiment, at least 355 contiguous amino acid residues.
In one embodiment, at least 360 contiguous amino acid residues.
In one embodiment, at least 365 contiguous amino acid residues.
In one embodiment, at least 370 contiguous amino acid residues.
In one embodiment, at least 375 contiguous amino acid residues.
In one embodiment, at least 380 contiguous amino acid residues.
In one embodiment, at least 385 contiguous amino acid residues.
In one embodiment, at least 390 contiguous amino acid residues.
In one embodiment, at least 395 contiguous amino acid residues.
In one embodiment, at least 400 contiguous amino acid residues.
In one embodiment, at least 405 contiguous amino acid residues.
In one embodiment, at least 410 contiguous amino acid residues.
In one embodiment, at least 415 contiguous amino acid residues.
In one embodiment, at least 420 contiguous amino acid residues.

Polypeptide fragments disclosed therein may be of any size that is compatible with the invention. They may range in size from the smallest specific epitope (*e.g*., about 6 amino acids) to a nearly full-length gene product (*e.g*., a single amino acid shorter than SEQ ID Nos: 2, 4, 6, 22, 24, 26, 28, 30, 32, 34, 36, 38, 40, 42 or 44).

The skilled person will appreciate that analogs of the polypeptides disclosed herein will also find use in the context of the present invention, i.e. as antigenic/immunogenic material. Thus, for instance proteins or polypeptides which include one or more additions, deletions, substitutions or the like are encompassed may be used in the present invention, in so far as this is encomparred by the appendant claims.

As used herein, "fragments", "analogs" or "derivatives" of the polypeptides include those polypeptides in which one or more of the amino acid residues are substituted with a conserved or non-conserved amino acid residue (preferably conserved) and which may be natural or unnatura...

These substitutions are those having a minimal influence on the secondary structure and hydropathic nature of the polypeptide. Preferred substitutions are those known in the art as conserved, i.e. the substituted residues share physical or chemical properties such as hydrophobicity, size, charge or functional groups. These include substitutions such as those described by Dayhoff, M. in Atlas of Protein Sequence and Structure 5, 1978 and by Argos, P. in EMBO J. 8, 779-785, 1989. For example, amino acids, either natural or unnatural, belonging to one of the following groups represent conservative changes:
ala, pro, gly, gln, asn, ser, thr, val;
cys, ser, tyr, thr;
val, ile, leu, met, ala, phe;
lys, arg, orn, his;
and phe, tyr, trp, his.
The preferred substitutions also include substitutions of D-enantiomers for the corresponding L-amino acids.

In one instance, preferred base substitutions are located where there is less sequence homology on Figures 31, 33 or 35.

In one instance, preferred amino acids substitutions are located where there is less sequence homology on Figures 32, 34 or 36.

The additional amino acid residues may be from a heterologous source or may be endogenous to the natural gene.

In an alternative approach, the analogs could be fusion polypeptides, incorporating moieties which render purification easier, for example by effectively tagging the desired polypeptide. It may be necessary to remove the "tag" or it may be the case that the fusion polypeptide itself retains sufficient antigenicity to be useful.

The percentage of homology is defined as the sum of the percentage of identity plus the percentage of similarity or conservation of amino acid type.

In one instance, analogs of polypeptides will have about 70% identity with those sequences illustrated in the figures or fragments thereof. That is, 70% of the residues are the same. In one embodiment, polypeptides for use in the invention will have greater than 95% identity. In a further embodiment, polypeptides will have greater than 98% identity. In a further embodiment, polypeptides will have greater than 99% identity. In a further instance, analogs of polypeptides will have fewer than about 20 amino acid residue substitutions, modifications or deletions and more preferably less than 10.

In one instance, analogs of polypeptides will have about 70% similarity with those sequences illustrated in the figures or fragments thereof. That is, 70% of the residues are the same. In a further instance, polypeptides will have greater than 80% similarity. In a further instance, polypeptides will have greater than 85% similarity. In a further instance, polypeptides will have greater than 90% similarity. In a further instance, polypeptides will have greater than 95% similarity. In a further instance, polypeptides will have greater than 98% similarity. In a further instance, polypeptides will have greater than 99% similarity. In a further instance, analogs of polypeptides will have fewer than about 20 amino acid residue substitutions, modifications or deletions and more preferably Less than 10.

In one instance, analogs of polyisomers will have about 70% homology with those sequences illustrated in the figures or fragments thereof. In a further instance, polypeptides will have greater than 80% homology. In a further instance, polypeptides will have greater than 85% homology. In a further instance, polypeptides will have greater than 90% homology. In a further instance, polypeptides will have greater than 95% homology. In a further instance, polypeptides will have greater than 98% homology. In a further instance, polypeptides will have greater than 99% homology. In a further instance, analogs of polypeptides will have fewer than about 20 amino acid residue substitutions, modifications or deletions and more preferably less than 10.

One can use a program such as the CLUSTAL program to compare amino acid sequences. This program compares amino acid sequences and finds the optimal alignment by inserting spaces in either sequence as appropriate. It is possible to calculate amino acid identity or homology for an optimal alignment. A program like BLASTx will align the longest stratch of similar sequences and assign a value to the fit. It is thus possible to obtain a comparison where several regions of similarity are found, each having a different score. Both types of identity analysis are contemplated in the present invention.

In an alternative approach, the analogs or derivatives could be fusion polypeptides, incorporating moieties which render purification easier, for example by effectively tagging the desired protein or polypeptide, it may be necessary to remove the "tag" or it may be the case that the fusion polypeptide itself retains sufficient antigenicity to be useful.

It is well known that it is possible to screen an antigenic polypeptide to identify epitopic regions, i.e. those regions which are responsible for the polypeptide's antigenicity or immunogenicity. Methods for carrying out such screening are well known in the art. Thus, the fragments disclosed herein should include one or more such epitopic regions or be sufficiently similar to such regions to retain their antigenic/immunogenic properties.

The disclosure also encompasses polypeptides having a lower degree of sequence identity, but having sufficient similarity so as to perform one or more of the functions or activities exhibited by the native polypeptides.

Thus, what is important for analogs, derivatives and fragments is that they possess at least a degree of the antigenicity/ immunogenicity of the protein or polypeptide from which they are derived.

Also included are polypeptides in which one or more of the amino acid residues includes a substituent group. These polypeptides include, *e.g.,* modified polypeptides. Know polypeptide modifications include, but are not limited to, glycosylation, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphatidylinositol, cross-linking, cyclization, disulfide bond formation, demethylation, formation of covalent crosslinks, formation of cystine, formation of pyroglutamate, formylation, gamma carboxylation, glycosylation, GPI anchor formatin, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination.

Such modifications are well-known to those of skill in the art and have been described in great detail in the scientific literature. Several particularly common modifications, glycosylation, lipid attachment, sulfation, gamma-carboxylation of glutamic acid residues, hydroxylation and ADP-ribosylation, for instance, are described in many basic texts, such as Proteins--Structure and Molecular Properties, 2nd ed., T.E. Creighton, W.H. Freeman and Company, New York (1993). Many detailed reviews are available on this subject, such as by Wold, F., Posttranslationail Covalent Modification of Proteins, B.C. Johnson, Ed., Academic Press, New York 1-12 (1983); Seifter et al. (1990) Meth. Enzymol. 182:626-646 and Rattan et al. (1992) Ann. N.Y. Acad. Sci. 663:48-62.

Also included are polypeptides which have fused thereto other compounds which alter the polypeptides biological or pharmacological properties i.e. polyethylene glycol (PEG) to increase half-life; leader or secretory amino acid sequences for ease of purification; prepro-and pro-sequences; and (poly) saccharides.

Furthermore, in those situations where amino acid regions are found to be polymorphic, it may be desirable to vary one or more particular amino acids to more effectively mimic the different epitopes of the different S. pyogenes strains.

Moreover, the polypeptides can be modified by terminal -NH₂ acylation (eg. by acetylation, or thioglycolic acid amidation, terminal carboxy amidation, e.g. with ammonia or methylamine) to provide stability, increased hydrophobicity for linking or binding to a support or other molecule.

Also contemplated are hetero and homo polypeptide multimers of the polypeptide fragments and analogues. These polymeric forms include, for example, one or more polypeptides that have been cross-linked with cross-linkers such as avidin/biotin, gluteraldehyde or dimethylsuperimidate. Such polymeric forms also include polypeptides containing two or more tandem or inverted contiguous sequences, produced from multicistronic mRNAs generated by recombinant DNA technology.

In a further instance, the present disclosure also relates to chimeric polypeptides which comprise one or more polypeptides or fragments or analogs thereof as defined in the figures of the present application.

In a further instance, the present disclosure also relates to chimeric polypeptides comprising two or more polypeptides comprising SEQ ID No : 2, 4, 6, 22, 24, 26, 28, 30, 32, 34, 36, 38, 40, 42, 44 or fragments or analogs thereof; provided that the polypeptides are linked as to form a chimeric polypeptide.

In a further instance, the present disclosure also relates to chimeric polypeptides comprising two or more polypeptides comprising SEQ ID No : 2, 4, 6, 22, 24, 26, 28, 30, 32, 34, 36, 38, 40, 42, 44 provided that the polypeptides are linked as to form a chimeric polypeptide.

Preferably, a fragment, analog or derivative of a polypeptide will comprise at least one antigenic region ie. at least one epitope.

In order to achieve the formation of antigenic polymers (i.e. synthetic multimers), polypeptides may be utilized having bishaloacetyl groups, nitroarylhalides, or the like, where the reagents being specific for thio groups. Therefore, the link between two mercapto groups of the different polypeptides may be a single bond or may be composed of a linking group of at least two, typically at least four, and not more than 16, but usually not more than about 14 carbon atoms.

In a particular embodiment, polypeptide fragments and analogs of the invention do not contain a starting residue, such as methionine (Met) or valine (Val). Preferably, polypeptides will not incorporate a leader or secretory sequence (signal sequence). The signal portion of a polypeptide may be determined according to established molecular biological techniques. In general, the polypeptide of interest may be isolated from a S. pyogenes culture and subsequently sequenced to determine the initial residue of the mature protein and therefore the sequence of the mature polypeptide.

It is understood that polypeptides can be produced and/or used without their start codon (methionine or valine) and/or without their leader peptide to favor production and purification of recombinant polypeptides. It is known that cloning genes without sequences encoding leader peptides will restrict the polypeptides to the cytoplasm of E. coli and will facilitate their recovery (Glick, B. R. and Pasternak, J. J. (1998) Manipulation of gene expression in prokaryotes. In "Molecular biotechnology: Principles and applications of recombinant DNA", 2nd edition, ASM Press, Washington DC, p. 109-143).

In another embodiment, the polypeptides for use in the invention may be lacking an N-terminal leader peptide, and/or a transmembrane domain and/or a C-terminal anchor domain.

The present disclosure further provides a fragment of the polypeptide comprising substantially all of the extra cellular domain of a polypeptide which has at least 70% identity, preferably 80% identity, more preferably 95% identity, to a sequence chosen from SEQ ID NOs: 2, 4, 6, 22, 24, 26, 28, 30, 32, 34, 36, 38, 40, 42, 44 or fragments or analogs thereof, over the entire length of said sequence.

According to another aspect of the disclosure, there are also provided (i) a composition of matter containing a polypeptide of the disclosure, together with a carrier, diluent or adjuvant; (ii) a pharmaceutical composition comprising a polypeptide of the disclosure and a carrier, diluent or adjuvant; (iii) a vaccine comprising a polypeptide of the disclosure and a carrier, diluent or adjuvant; (iv) a method for inducing an immune response against S. pyogenes, in a host, by administering to the host, an immunogenically effective amount of a polypeptide of the disclosure to elicit an immune response, e.g., a protective immune response to S. pyogenes; and particularly, (v) a method for preventing and/or treating a S. pyogenes infection, by administering a prophylactic or therapeutic amount of a polypeptide of the disclosure to a host in need.

According to another aspect of the disclosure, there are also provided (i) a composition of matter containing a polynucleotide of the disclosure, together with a carrier, diluent or adjuvant; (ii) a pharmaceutical composition comprising a polynucleotide of the disclosure and a carrier, diluent or adjuvant; (iii) a method for inducing an immune response against S. pyogenes, in a host, by administering to the host, an immunogenically effective amount of a polynucleotide of the disclosure to elicit an immune response, e.g., a protective immune response to S. pyogenes; and particularly, (iv) a method for preventing and/or treating a S. pyogenes infection, by administering a prophylactic or therapeutic amount of a polynucleotide of the disclosure to a host in need.

Before immunization, the polypeptides can also be coupled or conjugated to carrier proteins such as tetanus toxin, diphtheria toxin, hepatitis B virus surface antigen, poliomyelitis virus VP1 antigen or any other viral or bacterial toxin or antigen or any suitable proteins to stimulate the development of a stronger immune response. This coupling or conjugation can be done chemically or genetically. A more detailed description of peptide-carrier conjugation is available in Van Regenmortel, M.H.V., Briand J.P., Muller S., Plaué S., "Synthetic Polypeptides as antigens" in Laboratory Techniques in Biochemistry and Molecular Biology, Vol. 19 (ed.) Burdou, R.H. & Van Knippenberg P.H. (1988), Elsevier New York.

According to another aspect, there are provided pharmaceutical compositions comprising one or more S. pyogenes polypeptides or chimeric polypeptides as defined in the claims in a mixture with a pharmaceutically acceptable carrier or diluent.

According to another aspect, there are provided pharmaceutical compositions comprising one or more S. pyogenes polypeptides or chimeric polypeptides in a mixture with a pharmaceutically acceptable adjuvant. Suitable adjuvants include (1) oil-in-water emulsion formulations such as MH59™, SAF™, Ribi™ ; (2) Freund's complete or incomplete adjuvant; (3) salts i.e. AlK(SO₄)₂, AlNa(SO₄)₂, AlNH₄(SO₄)₂, Al(OH)₃, AlPO₄, silica, kaolin; (4) saponin derivatives such as Stimulon™ cr particles generated therefrom such as ISCOMs (immunostimulating complexes); (5) cytokines such as interleukins, interferons, macrophage colony stimulating factor (M-CSF), tumor necrosis factor (TNF) ; (6) other substances such as carbon polynucleotides i.e. poly IC and poly AU, detoxified cholera toxin (CTB)and E.coli heat labile toxin for induction of mucosal immunity; and (7) liposomes. A more detailed description of adjuvants is available in a review by M.Z.I Khan et al. in Pharmaceutical Research, vol. 11, No. 1 (1994) pp2-11, and also in another review by Gupta et al., in Vaccine, Vol. 13, No. 14, pp1263-1276 (1995) and in WO 99/24578. Preferred adjuvants include QuilA™, QS21™, Alhydrogel™ and Adjuphos™.

Pharmaceutical compositions of the invention may be administered parenterally by injection, rapid infusion, nasopharyngeal absorption, dermoabsorption, or buccal or oral.

The term pharmaceutical composition is also meant to include antibodies. In accordance with the present invention, there is also provided the use of one or more antibodies having binding specificity for the polypeptides of the present invention for the treatment or prophylaxis of streptococcus infection and/or diseases and symptoms mediated by streptococcus infection.

Pharmaceutical compositions of the invention are used for the prophylaxis of S. pyogenes infection and/or diseases and symptoms mediated by S. pyogenes infection as described in Manual of Clinical Microbiology, P. R. Murray (Ed, in chief), E. J. Baron, M. A. Pfaller, F. C. Tenover and R. H. Yolken. ASM Press, Washington, D. C. seventh edition, 1999,1773p.

Pharmaceutical compositions are for use in the prophylactic or therapeutic treatment or of many diverse infection types, including pharyngitis, erysipelas and impetigo, scarlet fever, and invasive diseases such as bacteremia and necrotizing fasciitis.

Pharmaceutical compositions may be used for the prophylactic or therapeutic treatment of Streptococcus infection and/or diseases and symptoms mediated by Streptococcus infection, in particular group A Streptococcus (Streptococcus pyogenes), group B Streptococcus (GBS or S. agalactiae), S. pneumoniae, S. dysgalactiae, S. uberis, S. nocardia as well as Staphylococcus aureus.

In one embodiment, pharmaceutical compositions of the invention are used for the prophylactic or therapeutic treatment of S. pyogenes infection. In a further embodiment, the S. pyogenes infection is nontypeable S. pyogenes.

Pharmaceutical compositions can also be specific or selective for one or more of the mentioned bacteria. For example, a composition can be selective or specific for a group A Streptococcus (Streptococcus pyogenes) and not react with group B Streptococcus (GBS or S. agalactiae), S. pneumoniae, S. dysgalactiae, S. uberis, S. nocardia nor Staphylococcus aureus.

The disclosure provides a method for prophylactic or therapeutic treatment of streptococcus infection in a host susceptible to streptococcus infection comprising administering to said host a prophylactic or therapeutic amount of a composition of the invention.

Also disclosed herein is a method for prophylactic or therapeutic treatment of S. pyogenes infection in a host susceptible to S. pyogenes infection comprising administering to said host a prophylactic or therapeutic amount of a composition of the invention.

As used in the present application, the term "host" includes mammals. In a further embodiment, the mammal is human.

In a particular embodiment, pharmaceutical compositions are administered to those hosts at risk of S. pyogenes infection such as neonates, infants, children, elderly and immunocompromised hosts.

In a particular embodiment, pharmaceutical compositions are administered to those hosts at risk of S. pyogenes infection such as adults.

Pharmaceutical compositions are preferably in unit dosage form of about 0.001 to 100 µg/kg (antigen/body weight) and more preferably 0.01 to 10 µg/kg and most preferably 0.1 to 1 µg/kg 1 to 3 times with an interval of about 1 to 6 week intervals between immunizations.

Pharmaceutical compositions are preferably in unit dosage form of about 0.1 µg to 10 mg and more preferably 1µg to 1 mg and most preferably 10 to 100 µg 1 to 3 times with an interval of about 1 to 6 week intervals between immunizations.

Also disclosed herein are polynucleotides encoding polypeptides characterized by the amino acid sequence comprising SEQ ID No : 2, 4, 6, 22, 24, 26, 28, 30, 32, 34, 36, 38, 40, 42, 44 or fragments or analogs thereof.

In one instance, polynucleotides are those illustrated in SEQ ID NO: 1, 3, 5, 21, 23, 25, 27, 29, 31, 33, 35, 37, 39, 41, 43 which may include the open reading frames (ORF). encoding the polypeptides disclosed herein.

Many types of variants of polynucleotides are encompassed by the disclosure including, *e.g.,* (i) one in which one or more of the nucleotides is substituted with another nucleotide, or which is otherwise mutated; or (ii) one in which one or more of the nucleotides is modified, *e.g*., includes a subtituent group; or (iii) one in which the polynucleotide is fused with another compound, such as a compound to increase the half-life of the polynucleotide; or (iv) one in which additional nucleotides are covalently bound to the polynucleotide, such a sequences encoding a leader or secretory sequence or a sequence which is employed for purification of the polypeptide. The additional nucleotides may be from a heterologous source, or may be endogenous to the natural gene.

Polynucleotide variants belonging to type (i) above include, *e.g.,* polymorphisms, including single nucleotide polymorphisms (SNPs), and mutants. Variant polynucleotides can comprise, *e.g.,* one or more additions, insertions, deletions, substitutions, transitions, transversions, inversions, or the like, or any combinations thereof.

Polynucleotide variants belonging to type (ii) above include, *e*.*g*., modifications such as the attachment of detectable markers (avidin, biotin, radioactive elements, fluorescent tags and dyes, energy transfer labels, energy-emitting labels, binding partner, etc.) or moieties which improve expression, uptake, cataloging, tagging, hybridization, detection, and/or stability. The polynucleotides can also be attached to solid supports, *e.g*., nitrocellulose, magnetic or paramagnetic micro spheres (*e.g.*, as described in U.S. Pat. No. 5,411,663; U.S. Pat. No. 5,543,289, for instance, comprising ferromagnetic, supermagnetic, paramagnetic, superparamagnetic, iron oxide and polysaccharide), nylon, agarose, diazotized cellulose, latex solid microspheres, polyacrylamides, etc., according to a desired method. See, e.g., U.S. Pat. Nos. 5,470,967; 5,476,925; 5,478,893.

Polynucleotide variants belonging to type (iii) above are well known in the art and include, *e.g*., various lengths of polyA⁺ tail, 5' cap structures, and nucleotide analogs, *e.g*., inosine, thionucleotides, or the like.

Polynucleotide variants belonging to type (i.v: above include, *e.g*., a variety of chimeric, hybrid or fusicn polynucleotides. For example, a polynucleotide can comprise a coding sequence and additional non-naturally occurring or haterologous coding sequence *(e.g.,* sequences coding for leader, signal, secretory, targeting, enzymatic, fluorescent, antibiotic resistance, and other functional or diagnostic peptides); or a coding sequence and non-coding sequences, *e.g.,* untranslated sequences at either a 5' or 3' end, or dispersed in the coding sequence.

It will be appreciated that the polynucleotide sequences illustrated in the figures may be altered with degenerate codons yet still encode the polypeptides of the disclosure. Accordingly the present disclosure further provides polynucleotides which hybridize to the polynucleotide sequences herein above described for the complement sequences thereof) having 70% identity between sequences. In one instance, at least 80% identity between sequences. In one instance, at least 85% identity between sequences. In one instance, at least 90% identity between sequences. In a further instance, polynucleotides are hybridizable under stringent conditions i.e. having at least 95% identity. In a further instance, more than 97% identity: In a further instance, more than 98% identity. In a further instance, more than 99% identity. In a further instance, polynucleotides are hybridizable under stringent conditions.

Suitable stringent conditions for hybridization can be readily determined by one of skilled in the art (see for example Sambrook et al., (1989) Molecular cloning : A Laboratory Manual, 2nd ed, Cold Spring Harbor, N.Y.; Current Protocols in Molecular Biology, (1999) Edited by Ausubel F.M. et al., John Wiley & Sons, Inc., N.Y.).

"Suitable stringent conditions", as used herein, means, for example, incubating a blot overnight *(e.g.,* at least 12 hours) with a long polynucleotide probe in a hybridization solution containing, *e.g.,* about 5X SSC, 0.5% SDS, 100 µg/ml denatured salmon sperm DNA and 50% formamide, at 42°C. Blots can be washed at high stringency conditions that allow, *e.g.,* for less than 5% bp mismatch (*e.g*., wash twice in 0.1X SSC and 0.1% SDS for 30 min at 65°C), thereby selecting sequences having, *e.g*., 95% or greater sequence identity.

Other non-limiting examples of suitable stringent conditions include a final wash at 65°C in aqueous buffer containing 30 mM NaCl and 0.5% SDS. Another example of suitable stringent conditions is hybridization in 7% SDS, 0.5 M NaPO₄, pH 7, 1 mM EDTA at 50°C, e.g., overnight, followed by one or more washes with a 1% SDS solution at 42°C. Whereas high stringency washes can allow for less than 5% mismatch, reduced or low stringency conditions can permit up to 20% nucleotide mismatch. Hybridization at low stringency can be accomplished as above, but using lower formamide conditions, lower temperatures and/or lower salt concentrations, as well as longer periods of incubation time.

According to another aspect of the disclosure there are also provided isolated polynucleotides comprising a sequence that hybridize under stringent conditions to either
a) a DNA sequence encoding a polypeptide or
b) the complement of a DNA sequence encoding a polypeptide; wherein said polypeptide comprises SEQ ID NO: 2, 4, 6, 22, 24, 26, 28, 30, 32, 34, 36, 38, 40, 42, 44 or fragments or analogs thereof;
polynucleotides that hybridize under stringent conditions to either
(a) a DNA sequence encoding a polypeptide or
(b) the complement of a DNA sequence encoding a polypeptide;
wherein said polypeptide comprises SEQ ID NO: 2, 4, 6, 22, 24, 26, 28, 30, 32, 34, 36, 38, 40, 42 or 44;
isolated polynucleotides comprising a sequence that hybridize under stringent conditions to either
a) a DNA sequence encoding a polypeptide or
b) the complement of a DNA sequence encoding a polypeptide; wherein said polypeptide has SEQ ID NO: 2, 4, 6, 22, 24, 26, 28, 30, 32, 34, 36, 38, 40, 42, 44 or fragments or analogs thereof;
polynucleotides that hybridize under stringent conditions to either
(a) a DNA sequence encoding a polypeptide or
(b) the complement of a DNA sequence encoding a polypeptide;
wherein said polypeptide has SEQ ID NO: 2, 4, 6, 22, 24, 26, 28, 30, 32, 34, 36, 38, 40, 42 or 44;
polynucleotides that hybridize under stringent conditions to either
(a) a DNA sequence encoding a polypeptide or
(b) the complement of a DNA sequence encoding a polypeptide;
wherein said polypeptide comprises at least 10 contiguous amino acid residues from a polypeptide comprising SEQ ID No: 2, 4, 6, 22, 24, 26, 28, 30, 32, 34, 36, 38, 40, 42, 44 or fragments or analogs thereof;
polynucleotides that hybridize under stringent conditions to either
(a) a DNA sequence encoding a polypeptide or
(b) the complement of a DNA sequence encoding a polypeptide;
wherein said polypeptide comprises at least 10 contiguous amino acid residues from a polypeptide comprising SEQ ID No : 2, 4, 6 22, 24, 26, 28, 30, 32, 34, 36, 38, 40, 42 or 44;
polynucleotides that hybridize under stringent conditions to either
a) a DNA sequence encoding a polypeptide or
b) the complement of a DNA sequence encoding a polypeptide;
wherein said polypeptide has at least 10 contiguous amino acid residues from a polypeptide having SEQ ID No : 2, 4, 6, 22, 24, 26, 28, 30, 32, 34, 36, 38, 40, 42, 44 or fragments or analogs thereof; and
polynucleotides that hybridize under stringent conditions to either
(a) a DNA sequence encoding a polypeptide or
(b) the complement of a DNA sequence encoding a polypeptide;
wherein said polypeptide has at least 10 contiguous amino acid residues from a polypeptide having SEQ ID No: 2, 4, 6 22, 24, 26, 28, 30, 32, 34, 36, 38, 40, 42 or 44.

In a further instance, polynucleotides are those illustrated in SEQ ID NO: 1, 3, 5, 21, 23, 25, 27, 29, 31, 33, 35, 37, 39, 41, 43 or fragments or analogs thereof encoding polypeptides of the invention.

In a further instance, polynucleotides are those illustrated in SEQ ID NO: 1, 3, 5, 21, 23, 25, 27, 29, 31, 33, 35, 37, 39, 41, 43 encoding polypeptides of the invention.

As will be readily appreciated by one skilled in the art, polynucleotides include both DNA and RNA.

The present disclosure also includes polynucleotides complementary to the polynucleotides described in the present application.

Polynucleotides encoding polypeptides as disclosed herein, or fragments, analogs or derivatives thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

Also disclosed is a process for producing polypeptides for use in the invention by recombinant techniques by expressing a polynucleotide encoding said polypeptide in a host cell and recovering the expressed polypeptide product.

Alternatively, the polypeptides can be produced according to established synthetic chemical techniques i.e. solution phase or solid phase synthesis of oligopeptides which are ligated to produce the full polypeptide (block ligation).

General methods for obtention and evaluation of polynucleotides and polypeptides are described in the following references: Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York; PCR Cloning Protocols, from Molecular Cloning to Genetic Engineering, Edited by White B.A., Humana Press, Totowa, New Jersey, 1997, 490 pages; Protein Purification, Principles and Practices, Scopes R.K., Springer-Verlag, New York, 3rd Edition, 1993, 380 pages; current Protocols in Immunology, Edited by Coligan J.E. et al., John Wiley & Sons Inc., New York.

The present disclosure provides a vector comprising a polynucleotide of the disclosure, wherein said DNA is operably linked to an expression control region.

The present disclosure provides a host cell transfected with the vector of the disclosure.

The present disclosure provides a process for producing a polypeptide comprising culturing a host cell of the disclosure under conditions suitable for expression of said polypeptide.

For recombinant production, host cells are transfected with vectors which encode the polypeptides of the invention, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York). Suitable promoters include but are not limited to LTR or SV40 promoter, E.coli lac, tac or trp promoters and the phage lambda P_{L} promoter. Vectors will preferably incorporate an origin of replication as well as selection markers i.e. ampicilin resistance gene. Suitable bacterial vectors include pET, pQE70, pQE60, pQE-9, pD10 phagescript, psiX174, pbluescript SK, pbsks, pNH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E.coli, Bacillus subtilis, Streptomyces; fungal i.e. Aspergillus niger, Aspergillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide i.e. using ammonium sulfate or ethanol precipitation, acid extraction, anion or cation exchange chromatography, phosphocellulose chromatography, hydrophobic interaction chromatography, hydroxylapatite chromatography and lectin chromatography. Final purification may be achieved using HPLC.

The polypeptides may be expressed with or without a leader or secretion sequence. In the former case the leader may be removed using post-translational processing (see US 4,431,739; US 4,425,437; and US 4,338,397) or be chemically removed subsequent to purifying the expressed polypeptide.

According to a further aspect, the S. pyogenes polypeptides of the invention may be used in a diagnostic test for Streptococcus infection, in particular S. pyogenes infection.

Several diagnostic methods are possible, as defined in the claims.

A method for the detection of antibody specific to a S. pyogenes antigen in a biological sample containing or suspected of containing said antibody may be performed as follows:
a) obtaining a biological sample from a host;
b) incubating one or more S. pyogenes polypeptides of the invention or fragments thereof with the biological sample to form a mixture; and
c) detecting specifically bound antigen or bound fragment in the mixture which indicates the presence of antibody specific to S. pyogenes.

One of skill in the art will recognize that this diagnostic test may take several forms, including an immunological test such as an enzyme-linked immunosorbent assay (ELISA), a radioimmunoassay or a latex agglutination assay, essentially to determine whether antibodies specific for the polypeptide are present in an organism.

The DNA sequences encoding polypeptides may also be used to design DNA probes for use in detecting the presence of S. pyogenes in a biological sample suspected of containing such bacteria. The detection method of this disclosure comprises:
a) obtaining the biological sample from a host;
b) incubating one or more DNA probes having a DNA sequence encoding a polypeptide of the invention or fragments thereof with the biological sample to form a mixture; and
c) detecting specifically bound DNA probe in the mixture which indicates the presence of S. pyogenes bacteria.

The DNA probes of this disclosure may also be used for detecting circulating S. pyogenes i.e. S. pyogenes nucleic acids in a sample, for example using a polymerase chain reaction, as a method of diagnosing S. pyogenes infections. The probe may be synthesized using conventional techniques and may be immobilized on a solid phase, or may be labelled with a detectable label. A preferred DNA probe for this application is an oligomer having a sequence complementary to at least about 6 contiguous nucleotides of the S. pyogenes polypeptides is dosal herein. In a further instance, the preferred DNA probe will be an oligomer having a sequence complementary to at least about 15 contiguous nucleotides of the S. pyogenes polypeptides disclosed herein. In a further instance, the preferred DNA probe will be an oligomer having a sequence complementary to at least about 30 contiguous nucleotides of the S. pyogenes polypeptides disclosed herein. In a further instance, the preferred DNA probe will be an oligomer having a sequence complementary to at least about 50 contiguous nucleotides of the S. pyogenes polypeptides disclosed herein.

Another diagnostic method for the detection of S. pyogenes in a host comprises:
a) labelling an antibody reactive with a polypeptide of the invention or fragment thereof with a detectable label;
b) administering the labelled antibody or labelled fragment to the host; and
c) detecting specifically bound labelled antibody or labelled fragment in the host which indicates the presence of S. pyogenes.

A further aspect of the disclosure the use of the S. pyogenes polypeptides of the invention as immunogens for the production of specific antibodies for the diagnosis and in particular the treatment of S. pyogenes infection. Suitable antibodies may be determined using appropriate screening methods, for example by measuring the ability of a particular antibody to passively protect against S. pyogenes infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the S. pyogenes polypeptides but is preferably specific for one.

According to one aspect, the present invention provides an antibody for use in treatment and/or prophylaxis of S. pyogenes infections, in so far as encompassed by the appendant claims.

A further aspect of the invention is antibodies for use in passive immunization, in so far or this is encompassed by the appendant claims. One could use the antibodies described in the present application.

Also disclosed herein is a method for immunization, whereby an antibody raised by a polypeptide of the invention is administered to a host in an amount sufficient to provide a passive immunization.

The use of a polynucleotide in genetic immunization will preferably employ a suitable delivery method or system such as direct injection of plasmid DNA into muscles [Wolf et al. H M G (1992) 1: 363; Turnes et al., Vaccine (1999), 17 : 2089; Le et al., Vaccine (2000) 18 : 1893 Alves et al., Vaccine (2001) 19 : 788], injection of plasmid DNA with or without adjuvants [Ulmer et al., Vaccine 1999) 18: 18; MacLaughlin et al., J. Control Release (1998) 56: 259; Hartikka et al., Gene Ther. (2000) 7: 1171-82; Benvenisty and Reshef, PNAS USA (1986) 83:9551; Singh et al., PNAS USA (2000) 97: 811], targeting cells by delivery of DNA complexed with specific carriers [Wa et al., J Biol Chem (1989) 264: 15985; Chaplin et al., Infect. Immun. (1999) 67: 6434], injection of plasmid complexed or encapsulated in various forms of liposomes [Ishii et al., AIDS Research and Human Retroviruses (1997) 13: 242; Perrie et al., Vaccine (2001) 29: 3301], administration of DNA with different methods of bombardment [Tang et al., Nuture (1992) 356: 152; Eisenbraun et al., DNA Cell Biol (1993) 12: 791; Chen et al., Vaccine (2001) 19: 2908], and administration of DNA with lived vectors [Tubulekas et al., Gene (1997) 190: 191; Pushko et al., Virology (1997) 239: 389; Spreng et al. FEMS (2000) 27: 299; Dietrich et al., Vaccine (2001) 19: 2506].

In a further embodiment, the invention provides the use of a pharmaceutical composition of the invention in the manufacture of a medicament for the prophylactic or therapeutic treatment of s. pyogenes infection, in so far as encompassed by the appendant claims.

In a further instance, the disclosure provides a kit comprising a polypeptide of the invention for detection or diagnosis of S. pyogenes infection.

Unless otherwise defined, all technical and scientific terms used herein have the same meaning as commonly understood by one of ordinary skill in the art to which this invention belongs. In case of conflict, the present specification, including definitions, will control. In addition, the materials, methods, and examples are illustrative only and not intended to be limiting.

### EXAMPLE 1

This example illustrates the cloning and molecular characteristics of **SHB-GAS-102** gene and corresponding polypeptide

The coding region of S. pyogenes SHB-GAS-102 (SEQ ID NO: 1) gene was amplified by PCR (Robocycler Gradient 96 Temperature cycler, Stratagene, La Jolla, CA) from genomic DNA of serotype M1 S. pyogenes strain ATCC700294 using the following oligonucleotide primers that contained base extensions for the additicn of restriction sites *N*deI (CATATG) and *Xho*I (CTCGAG): DMAR2174 and DMAR2175, which are presented in Table 1. PCR products were purified from agarose gel using a QIAquick gel extraction kit from QIAgen following the manufacturer's instructions (Chatsworth, CA), and digested with *Nde*I and *Xho*I (Amersham Biosciences Inc, Baie d'Urfé, Canada). The pET-19b(+) vector (Novagen, Madison, WI) was digested with *Nde*I and *Xho*I and purified from agarose gel using a QIAquick gel extraction kit from QIAgen (Chatsworth, CA). The *Nde*I-*Xho*I PCR products were ligated to the *Nde*I-*Xho*I pET-19b(+) expression vector. The ligated products were transformed into E. coli strain DH5α 5 [φ80d*lac*ZΔM15 Δ(*lac*ZYA-*arg*F)U169 *end*A1 *rec*A1 *hsd*R17(r_{K}-m_{K}+) deoR thi-1 supE44 λ⁻*gyr*A96 *rel*A1] (Gibco BRL, Gaithersburg, MD) according to the method of Simanis (Hanahan, D. DNA Cloning, 1985, D.M. Glover (ed), pp. 109-135). Recombinant pET-19b(+) plasmid (rpET19b(+)) containing SHB-GAS-102 gene was purified using the GenElute plasmid kit (Sigma-Aldrich Company Ltd, MO) and DNA insert was sequenced (Taq Dye Deoxy Terminator Cycle Sequencing kit, ABI, Foster City, CA).

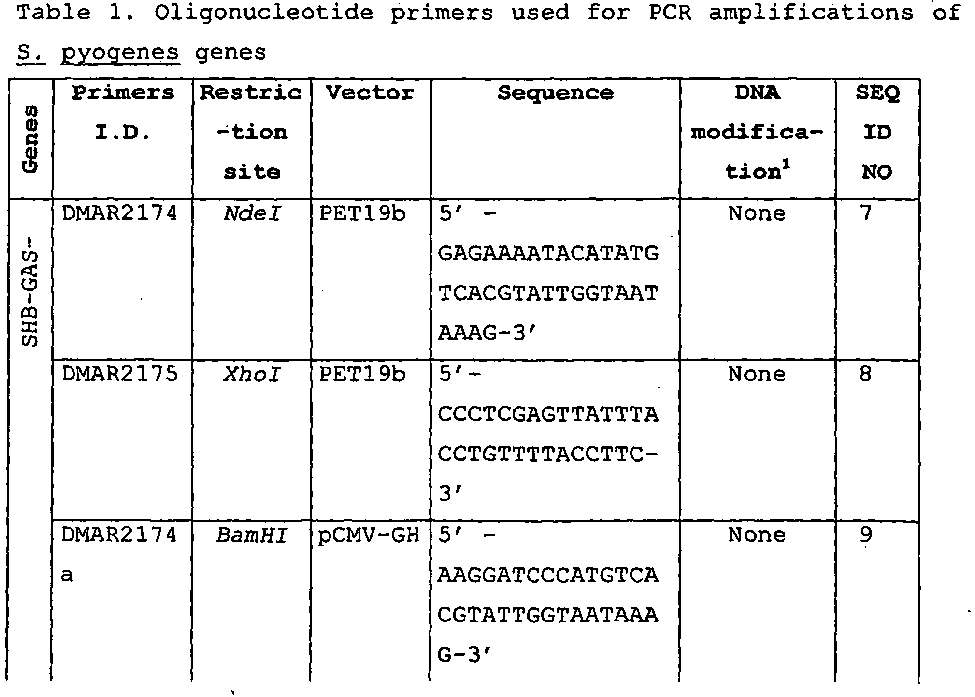

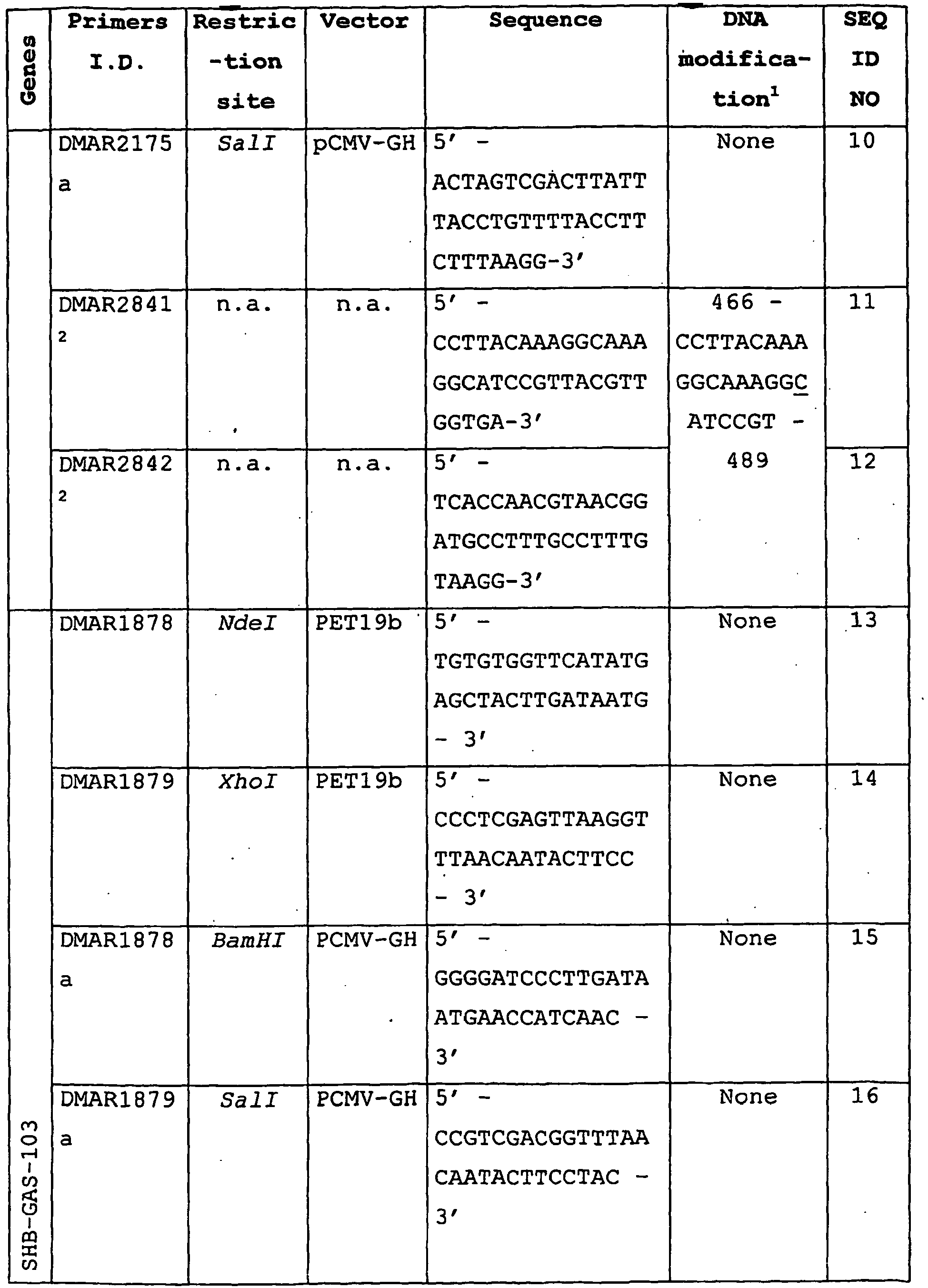

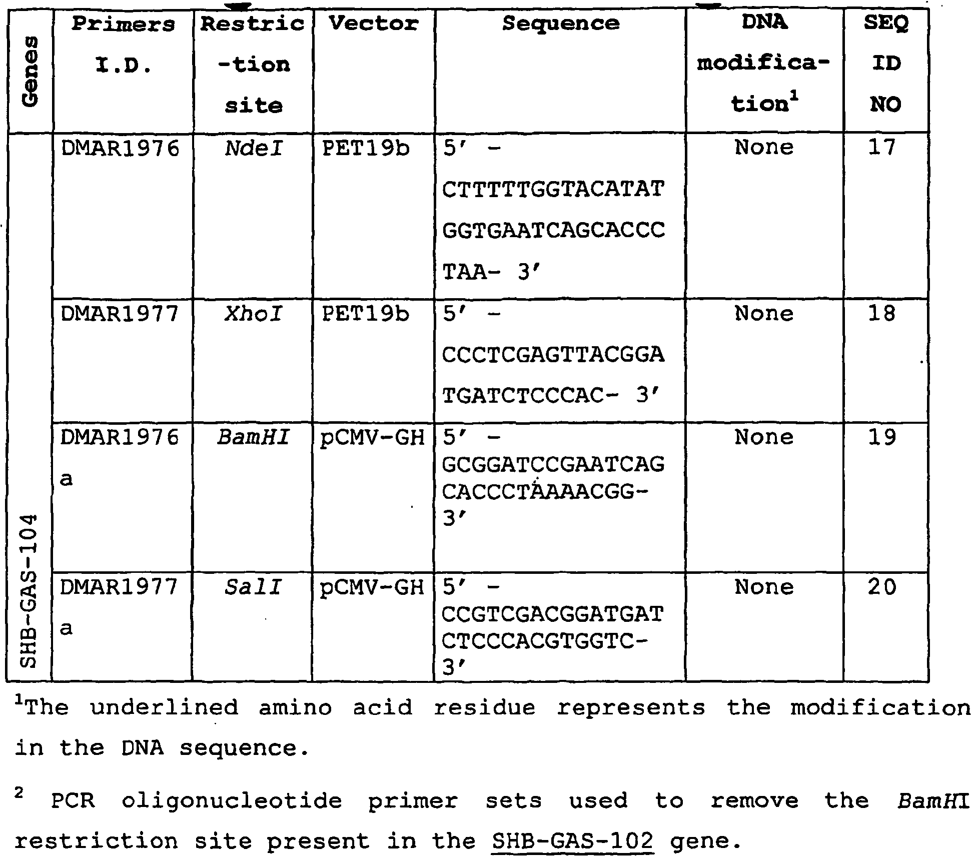

It was determined that the 537-bp including a stop codon (TAA) open reading frame (ORF) of SHB-GAS-102 encodes a 178 amino-acid residues polypeptide with a predicted pI of 9.55 and a predicted molecular mass of 19,431.0 Da. Analysis of the predicted amino acid residues sequence (SEQ ID NO: 2) using the Spscan sofware (Wisconsin Sequence Analysis Package; Genetics Computer Group) did not reveal the existence of a signal peptide.

To confirm the presence by PCR amplification of SHB-GAS-102 (SEQ ID NO :1) gene, the following 4 serologically distinct S. pyogenes strains were used: the serotype M1 S. pyogenes strain ATCC700294, the serotype M3 S. pyogenes strain ATCC12384, and the serotype M18 S. pyogenes strain ATCC12357 were obtained from the American Type Culture Collection (Rockville, MD), and the serotype M6 S. pyogenes SPY67 clinical isolate was provided by the Centre de recherche en infectiologie du Centre hospitalier de l'Université Laval, Sainte-Foy, Canada. Chromosomal DNA was isolated from each strain as previously described (Jayarao BM et al. 1991. J. Clin. Microbiol. 29:2774-2778). SHB-GAS-102 (SEQ ID NO :1) gene was amplified by PCR (Robocycler Gradient 96 Temperature cycler) from the genomic DNA purified from the 4 S. pyogenes strains using the oligonucleotide primers DMAR2174 and DMAR2175 (Table 1). PCR was performed with 35 cycles of 45 sec at 95°C, 45 sec at 45°C and 75 sec at 72°C and a final elongation period of 10 min at 72°C. The PCR products were size fractionated in 1% agarose gels and were visualized by ethidium bromide staining. The results of these PCR amplifications are presented in Table 2. The analysis of the amplification products revealed that SHB-GAS-102 (SEQ ID NO :1) gene was present in the genome of all of the 4 S. pyogenes strains tested. These PCR data presented in the previous paragraphs clearly demonstrated that the SHB-GAS-102 gene is highly conserved among streptococcal strains.

**Table 2. Identification of S. pyogenes genes by PCR amplification**

| **Strain Identification** | **Identification by PCR amplification of** | | |
|---|---|---|---|
| | SHB-GAS-102 | SHB-GAS-103 | SHB-GAS-104 |
| ATCC700294 (M1) | + | + | + |
| ATCC12384 (M3) | + | + | + |
| SPY67 (M6) | + | + | + |
| ATCC12357 (M18) | + | + | + |

In order to evaluate the distribution of the SHB-GAS-102 polypeptide among S. pyogenes isolates, the reactivity of pooled mouse anti-SHB-GAS-102 sera to a collection of 13 strains of S. pyogenes representing 13 M serotypes (Table 3) was tested by immunoblots. These sera were collected from mice immunized three times at two-week intervals with 20 µg of purified recombinant SHB-GAS-102 polypeptides mixed with Quil A adjuvant. The recombinant SHB-GAS-102 polypeptide was produced and purified as described in Example 6. The S. pyogenes cells used for this study were prepared following this protocol: bacteria were grown in Todd Hewitt (TH) broth with 0.5% Yeast extract and 1% peptone extract overnight at 37°C in a 8% CO₂ atmosphere. The bacterial suspension was adjusted to an OD₆₀₀ₙₘ of 1.0 and the S. pyogenes cells were fixed with 0.3% formaldehyde in PBS buffer for 18 h at 4°C. After incubation, this solution was treated with mutanolysin (7.5U/500µl) for 30 min at 37°C, and boiled for 5 min in SDS-PAGE sample buffer. Antibodies present in the pooled mouse sera recognized the SHB-GAS-102 polypeptide band (≈22 kDa) in all the 13 S. pyogenes preparations tested (Table 3). In order to confirm the nature of the polypeptide band, anti-SHB-GAS-102 sera were adsorbed with 12.5 µg of purified recombinant SHB-GAS-102 polypeptides (overnight, 4°C). As expected, this adsorbed sera did not recognize, in the S. pyogenes whole-cell extract applied onto the gel, the polypeptide band at ≈22 kDa confirming that this polypeptide band was the SHB-GAS-102 polypeptide.

These data as well as the data presented in the previous paragraphs clearly demonstrated that the SHB-GAS-102 gene and the SHB-GAS-102 polypeptide are highly conserved among streptococcal strains.

**Table 3. Identification of S. pyogenes polypeptides by immunoblots with pooled mouse anti-sera raised against recombinant SHB-GAS-102, SHB-GAS-103 and SHB-104 polypeptides¹.**

| **Strain identification (M serotype)** | **Identification by immunoblot of** | | |
|---|---|---|---|
| | SHB-GAS-102 | SHB-GAS-103 | SHB-GAS-104 |
| ATCC700294 (M1) | + | + | + |
| Spy68 (M2) | + | + | + |
| ATCC12384 (M3) | + | + | + |
| Spy71 (M4) | + | + | + |
| Spy70 (M5) | + | + | + |
| Spy67 (M6) | + | + | + |
| Spy88 (M11) | + | + | + |
| Spy95 (M12) | + | + | + |
| ATCC12357 (M18) | + | + | + |
| Spy73 (M22) | + | + | + |
| Spy91 (M28) | + | + | + |
| Spy99 (M58) | + | + | + |
| Spy87 (M77) | + | + | + |

| | | | |
|---|---|---|---|
| ¹Sera were collected from mice immunized three times at two-week intervals with 20 µg of purified recombinant polypeptides mixed with Quil A adjuvant. Sera were diluted 1/1000 to perform the immunoblots. | | | |

### EXAMPLE 2

This example illustrates the cloning and molecular characteristics of SHB-GAS-103 gene and corresponding polypeptide

The coding region of S. pyogenes SHB-GAS-103 gene (SEQ ID NO: 3) was amplified by PCR (Robocycler Gradient 96 Temperature cycler, Stratagene, LaJolla, Ca) from genomic DNA of serotype M1 S. pyogenes strain ATCC700294 using the following oligos that contained base extensions for the addition of restruction sites *Nd*eI (CATATG) and *Xho*I (CTCGAG): DMAR1878 and DMAR1879, which are presented in Table 1. The methods used for cloning SHB-GAS-103 into an expression vector and sequencing are similar to the methods described in Example 1.

It was determined that the 1269-bp including a stop codon (TAA) open reading frame (ORF) of SHB-GAS-103 encodes a 422 amino-acid-residues polypeptide with a predicted pI of 9.11 and a predicted molecular mass of 46,605.3 Da. Analysis of the predicted amino acid residues sequence (SEQ ID NO: 4) using the Spscan sofware (Wisconsin Sequence Analysis Package; Genetics Computer Group) suggested the existence of a 27 amino acid residues signal peptide (MFQLRKKMTRKQLALLSAGVLTCVVGG).

The SHB-GAS-103 gene was shown to be present after PCR amplification using the oligonucleotide primers DMAR1878 and DMAR1879 in the 4 serologically S. pyogenes strains tested (Table 2). In addition, the SHB-GAS-103 polypeptide was shown to be present in all the 13 S. pyogenes isolates tested by immunoblots as described in Example 1 (Table 3). Indeed, antibodies present in the pooled mouse sera recognized the SHB-GAS-103 polypeptide band (≈52 kDa) in all the 13 S. pyogenes preparations tested (Table 3). In order to confirm the nature of the polypeptide band, anti-SHB-GAS-103 sera were adsorbed with 12.5 µg of purified recombinant SHB-GAS-103 polypeptides (overnight, 4°C). As expected, this adsorbed sera did not recognize, in the S. pyogenes whole-cell extract applied onto the gel, the polypeptide band at ≈52 kDa confirming that this polypeptide band was the SHB-GAS-103 polypeptide.

These data presented in the previous paragraphs clearly demonstrated that the SHB-GAS-103 gene and the SHB-GAS-103 polypeptide are highly conserved among streptococcal strains.

### EXAMPLE 3

This example illustrates the cloning and molecular characteristics of SHB-GAS-104 gene and corresponding polypeptide

The coding region of S. pyogenes SHB-GAS-104 gene (SEQ ID NO: 5) was amplified by PCR (Robocycler Gradient 96 Temperature cycler, Stratagene, LaJolla, Ca) from genomic DNA of serotype M1 S. pyogenes strain ATCC700294 using the following oligos that contained base extensions for the addition of restriction sites NdeI (CATATG) and XhoI (CTCGAG): DMAR1976 and DMAR1977, which are presented in Table 1. The methods used for cloning SHB-GAS-104 into an expression vector and sequencing are similar to the methods described in Example 1.

It was determined that the 885-bp including a stop codon (TAA) open reading frame (ORF) of SHB-GAS-104 encodes a 294 amino-acid-residues polypeptide with a predicted pI of 5.83 and a predicted molecular mass of 33,381.9 Da. Analysis of the predicted amino acid residues sequence (SEQ ID NO: 6) using the Spscan sofware (Wisconsin Sequence Analysis Package; Genetics Computer Group) suggested the existence of a 19 amino acid residues signal peptide (MIKRCKGIGLALMAFFLVA).

The SHB-GAS-104 gene was shown to be present after PCR amplification using the oligonucleotide primers DMAR1976 and DMAR1977 in the 4 serologically S. pyogenes strains tested (Table 2). The methods used for PCR amplification of the SHB-GAS-104 gene were similar to the methods presented in Example 1.

In addition, the distribution of the SHB-GAS-104 polypeptide among S. pyogenes isolates have been evaluated as described in Example 1 using a S. pyogenes extract prepared by the following method: bacteria were grown in TH broth with 0.5% Yeast extract and 1% peptone extract overnight at 37°C in a 8% CO₂ atmosphere. The bacterial suspension was adjusted to an OD₆₀₀ₙₘ of 0.8. After centrifugation, the bacterial pellet was resuspended in 500 µl of extraction buffer (0.1 M Tris-HCl pH 7.6) and pelleted by centrifugation. The pellet was frozen for 10 min at - 80°C and thawed at 37°C for 5 min. The freeze-thaw cycle was repeated three times. Bacterial pellet was resuspended in 500 µl of extraction buffer and sonicated 8 x 15 sec. Samples were centrifuged (14 000 X g) at 4°C for 15 min and supernatant was boiled for 5 min in SDS-PAGE sample buffer. Antibodies present in the pooled mouse anti-SHB-GAS-104 sera recognized the SHB-GAS-104 polypeptide band (≈37.5 kDa) in all of the 13 S. pyogenes preparations tested (Table 3). In order to confirm the nature of the polypeptide band, anti-SHB-GAS-104 sera were adsorbed with 12.5 µg of purified recombinant SHB-GAS-104 polypeptide (overnight, 4°C). As expected, this adsorbed serum did not recognize, in the S . pyogenes extract applied onto the gel, the polypeptide band at ≈37.5 kDa confirming that this polypeptide band was the SHB-GAS-104 polypeptide.

These PCR data presented in the previous paragraphs clearly demonstrated that the SHB-GAS-104 gene is highly conserved among streptococcal strains.

### EXAMPLE 4

This example illustrates the cloning of S. pyogenes genes in CMV plasmid pCMV-GH.

The DNA coding regions of S. pyogenes polypeptides were inserted in phase downstream of a human growth hormone (hGH) gene which was under the transcriptional control of the cytomegalovirus (CMV) promotor in the plasmid vector pCMV-GH (Tang et al., Nature, 1992, 356 :152). The CMV promotor is a non functional plasmid in E. coli cells but active upon administration of the plasmid in eukaryotic cells. The vector also incorporated the ampicillin resistance gene.

In order to remove the *BamH*I site into the SHB-GAS-102 gene, mutagenesis experiments using the Quickchange Site-Directed Mutagenesis kit from Stratagene were performed according to the manufacturer's recommendations. The oligonucleotides DMAR2841 and DMAR2842 (Table 1) and the SHB-GAS-102 gene cloned into pET vector as DNA template were used to perform the mutagenesis experiments. The modification on the SHB-GAS-102 gene generated by site-directed mutagenesis is presented in the Table 1.

The coding regions of modified SHB-GAS-102, SHB-GAS-103 (SEQ ID NO: 3), and SHB-GAS-104 (SEQ ID NO: 5) genes were amplified by PCR (Robocycler Gradient 96 Temperature cycler, Stratagene, LaJolla, Ca) from pET vector containing the modified SHB-GAS-102 gene or the genomic DNA of serotype M1 S. pyogenes strain ATCC700294 (for SHB-GAS-103 and SHB-GAS-104 genes) using oligonucleotide primers that contained base extensions for the addition of restriction sites *BamH*I (GGATCC) and *Sal*I (GTCGAC) which are described in Table 1. The PCR products were purified from agarose gel using a QIAquick gel extraction kit from QIAgen (Chatsworth, CA) and digested with restriction enzymes (Amersham Biosciences Inc, Baie d'Urfé, Canada). The pCMV-GH vector (Laboratory of Dr. Stephen A. Johnston, Department of Biochemistry, The University of Texas, Dallas, Texas) was digested with *BamH*I and *Sal*I and purified from agarose gel using the QIAquick gel extraction kit from QIAgen (Chatsworth, CA). The *BamH*I-*Sal*I DNA fragments were ligated to the *BamH*I-*Sal*I pCMV-GH vector to create the hGH-SHB-GAS-102, hGH-SHB-GAS-103, and hGH-SHB-GAS-104 fusion polypeptides under the control of the CMV promoter. The ligated products were transformed into E. coli strain DH5α [φ80d*l*acZΔM15 Δ(*lac*ZYA-*arg*F) U169 *end*A1 *rec*A1 *hsd*R17 (r_{K}-m_{K}+) *deo*R *thi*-1 *sup*E44 λ⁻*gyr*A96 *rel*A1] (Gibco BRL, Gaithersburg, MD) according to the method of Simanis (Hanahan, D. DNA Cloning, 1985, D.M. Glover (ed), pp. 109-135). The recombinant pCMV plasmids were purified using the GenElute plasmid kit (Sigma-Aldrich company Ltd, MO) and the nucleotide sequences of the DNA inserts were verified by DNA sequencing.

### EXAMPLE 5

This example illustrates the use of DNA to elicit an immune response to S. pyogenes polypeptide antigens.

Groups of 8 female BALB/c mice (Charles River, St-Constant, Québec, Canada) were immunized by intramuscular injection of 100 µl three times at two- or three-week intervals with 50 µg of recombinant pCMV-GH encoding modified SHB-GAS-102, SHB-GAS-103 (SEQ ID NO: 3), and SHB-GAS-104 (SEQ ID NO: 5) genes in presence of 50 µg of granulocyte-macrophage colony-stimulating factor (GM-CSF)- expressing plasmid pCMV-GH-GM-CSF (Laboratory of Dr. Stephen A. Johnston, Department of Biochemistry, The University of Texas, Dallas, Texas). As control, groups of mice were injected with 50 µg of pCMV-GH in presence of 50 µg of pCMV-GH-GM-CSF. Blood samples were collected from the orbital sinus prior to each immunization and seven days following the third injection and serum antibody responses were determined by ELISA using the corresponding His-tagged labeled S. pyogenes recombinant polypeptides as coating antigens. The production and purification of these His-tagged labeled S. pyogenes recombinant polypeptides are presented in Example 6.

### EXAMPLE 6

This example illustrates the production and purification of S. pyogenes recombinant polypeptides.

The recombinant pET-19b(+)plasmids with SHB-GAS-102 (SEQ ID NO: 1), SHB-GAS-103 (SEQ ID NO: 3), and SHB-GAS-104 (SEQ ID NO: 5) genes were used to transform by electroporation (Gene Pulser II apparatus, BIO-RAD Labs, Mississauga, Canada) E. coli strains BL21 (DE3) (F⁻ompT hsdS_{B} (r⁻_{B}m⁻_{B}) gal dcm (DE3)) or BL21 star (DE3) (F⁻ompT hsdS_{B} (r⁻_{B}m⁷_{B}) gal dcm rne131 (DE3)) (Novagen, Madison, WI). In these strains of E. coli, the T7 promotor controlling expression of the recombinant polypeptide is specifically recognized by the T7 RNA polymerase (present on the λDE3 prophage) whose gene is under the control of the lac promotor which is inducible by isopropyl-ß-d-thiogalactopyranoside (IPTG). The transformants BL21(DE3)/rpET or BL21 star (Des)/rpET were grown at 37°C with agitation at 250 rpm in LB broth (peptone 10g/L, yeast extract 5g/L, NaCl 10g/L) containing 100 µg of carbenicillin (Sigma-Aldrich Canada Ltd., Oakville, Canada) per ml until the A₆₀₀ reached a value of 0.6. In order to induce the production of His-tagged S. pyogenes recombinant polypeptides, the cells were incubated for 2 additional hours in the presence of IPTG at a final concentration of 1 mM. Induced cells from a 500 ml culture were pelleted by centrifugation and frozen at -70°C.

The purification of the recombinant polypeptides from the soluble fraction of IPTG-induced BL21 (DE3) /rpET19b(+) or BL21 star(DE3)/rpETl9b(+) was done by affinity chromatography based on the properties of the His●Tag sequence (10 consecutive histidine residues) to bind to divalent cations (Ni²⁺) immobilized on the His●Bind metal chelation resin. Briefly, the pelleted cells obtained from a 400 mL culture induced with IPTG was resuspended in lysis buffer (20 mM Tris, 500 mM NaCl, 10 mM imidazole, pH 7.9), sonicated and centrifuged at 12,000 X g for 20 min to remove debris. The supernatant was incubated with Ni-NTA agarose resin (QIAgen) for 45 min at 4°C. The S. pyogenes recombinant polypeptides were eluted from the resin with a solution of 250 mM imidazole-500mM NaCl-20 mM Tris, pH 7.9. The removal of the salt and imidazole from the samples was done by dialysis against PBS buffer overnight at 4°C. The amount of recombinant polypeptide was estimated by MicroBCA (Pierce, Rockford, Ill.).

### EXAMPLE 7

This example illustrates the accessibility to antibodies of the S. pyogenes recombinant polypeptides at the surface of intact streptococcal cells.

Bacteria were grown in Todd Hewitt (TH) broth (Difco Laboratories, Detroit, MI) with 0.5% Yeast extract (Difco Laboratories) and 1% peptone extract (Merck, Darmstadt, Germany) at 37°C in a 8% CO₂ atmosphere to give an OD₆₀₀ₙₘ of 0.600 (∼10⁹ CFU/ml). Dilutions of anti-SHB-GAS-102, anti-SHB-GAS-103, anti-SHB-GAS-104, or control sera were then added and allowed to bind to the cells, which were incubated for 2 h at 4°C. Samples were washed 2 times in blocking buffer [phosphate-buffered saline (PBS) containing 2% bovine serum albumin (BSA)], and then 0.5 ml of goat fluorescein (FITC)-conjugated anti-mouse IgG + IgM diluted in blocking buffer was added. After an additional incubation of 60 min at room temperature, samples were washed 2 times in blocking buffer and fixed with 0.3 % formaldehyde in PBS buffer for 18-24 h at 4°C. Cells were kept in the dark at 4°C until analyzed by flow cytometry (Epics® XL; Beckman Coulter, Inc.). Ten thousands intact S. pyogenes cells were analyzed per sample.

### EXAMPLE 8

This example illustrates the protection against fatal S. pyogenes infection induced by passive immunization of mice with rabbit hyper-immune sera.

New Zealand rabbit (Charles River) was immunized subcutaneously three times at 3-week intervals at multiple sites with 100 µg of His-tagged recombinant SHB-GAS-102 protein in the presence of Freund's incomplete adjuvant (Gibco-BRL). Blood samples were collected three weeks after the third injection. The antibodies present in the serum were partially purified by precipitation using 40% saturated ammonium sulfate solution. Groups of 6 female Balb/c mice (Charles River) were injected intravenously with 500 µl of partially purified serum collected from a rabbit immunized with the recombinant SHB-GAS-102 protein, or a rabbit immunized with an unrelated control recombinant protein. Eighteen hours later the mice were challenged with approximately 2x10⁷ CFU of the type 3 S. pyogenes strain ATCC12384. Samples of the S. pyogenes challenge inoculum were plated on blood agar plates to determine the CFU and to verify the challenge dose. Deaths were recorded for a period of 8 days.

As presented in Table 4, 67% (4/6) mice immunized with partially purified serum collected from rabbit immunized with the recombinant SHB-GAS-102 protein were protected against a lethal challenge. On the contrary, immunization of mice withy serum collected from rabbit immunized with an irrelevant protein did not confer such protection (Table 4).

**Table 4. Ability of SHB-GAS-102-specific antibodies to elicit passive protection against S. pyogenes strain ATCC12384 (M3)**

| Group | No. mice surviving | % survival |
|---|---|---|
| SHB-GAS-102-specific antibodies | 4/6 | 67 % |
| Unrelated protein-specific antibodies | 0/6 | 0 % |

### EXAMPLE 9

This example illustrates the protection of mice against fatal S. pyogenes infection induced by immunization with recombinant polypeptides.

Groups of female Balb/c mice (Charles River) were immunized subcutaneously three times at two-week intervals with 20 µg of affinity purified SHB-GAS-102, SHB-GAS-103, or SHB-GAS-104 His-tagged recombinant polypeptides in presence of 10 ug of QuilA adjuvant (Cedarlane Laboratories Ltd, Hornby, Canada) or, as control, with QuilA adjuvant alone in PBS or with 20 µg of irrelevant polypeptide in presence of QuilA. Blood samples were collected from the orbital sinus on day 1, 14 and 28 prior to each immunization and 14 days (day 42) following the third injection. One week later the mice were challenged with approximately 4-8x10⁶ CFU of the type 3 S. pyogenes strain ATCC12384. Samples of the S. pyogenes challenge inoculum were plated on blood agar plates to determine the CFU and to verify the challenge dose. Deaths were recorded for a period of 7 days.

As presented in Table 5, more than 83% (10/12) mice immunized with SHB-GAS-102 polypeptide were protected against a lethal challenge. Mice immunized with SHB-GAS-103 and SHB-GAS-104 were also protected against a lethal challenge (7/12). On the contrary, immunization of mice with adjuvant only or with an irrelevant protein did not confer such protection (Table 5).

**Table 5. Ability of recombinant SHB-GAS-102, SHB-GAS-103, and SHB-GAS-104 polypeptides to elicit protection against S. pyogenes strain ATCC12384 (M3)**

| **Groups** | **No. mice surviving** | **% survival** |
|---|---|---|
| 20µg of SHB-GAS-102 polypeptide + 10µg of QuilA | 10/12 | 83 |
| 20µg of SHB-GAS-103 polypeptide + 10µg of QuilA | 7/12 | 58 |
| 20µg of SHB-GAS-104 polypeptide + 10µg of QuilA | 7/12 | 58 |
| 20µg of irrelevant polypeptide + 10µg of QuilA | 2/12 | 17 |
| 10 µg of QuilA in PBS | 3/12 | 25 |

## Claims

1. A pharmaceutical composition comprising:
(a) a polypeptide selected from (i) a polypetide that comprises an amino acid sequence at least 95% identical to the amino acid sequence set forth in Figure 2; and (ii) a polypeptide comprising an immunogenic polypeptide fragment consisting of at least 10 contiguous amino acids of the amino acid sequence set forth in Figure 2,
wherein the polypeptide is capable of raising antibodies that specifically bind to a protein consisting of the amino acid sequence set forth in Figure 2, and wherein the polypeptide is capable of eliciting an immune response against *Streptococcus pyogenes,* and
(b) a pharmaceutically acceptable carrier or diluent,
wherein the pharmaceutical composition is for use in the prophylactic or therapeutic treatment of *Streptococcus pyogenes* infection.

2. The pharmaceutical composition according to claim 1 wherein the polypeptide comprises the amino acid sequence set forth in Figure 2.

3. The pharmaceutical composition according to claim 2 wherein the polypeptide lacks the N-terminal methionine residue.

4. The pharmaceutical composition according to any one of claims 1-3 wherein the composition further comprises a pharmaceutically acceptable adjuvant.

5. The pharmaceutical composition according to any one of claims 1-3 wherein the polypeptide is produced recombinantly by a method comprising culturing a host cell transfected with a vector that comprises a polynucleotide encoding the polypeptide, wherein the polynucleotide is operably linked to an expression control region.

6. A pharmaceutical composition comprising (a) an antibody, or antigen-binding fragment thereof, that specifically binds to a polypeptide consisting of the sequence set forth in Figure 2 wherein the antibody, or antigen-binding fragment thereof, is capable of protecting a host from a *Streptococcus pyogenes* infection, and (b) a pharmaceutically acceptable carrier or diluent, for use in the prophylactic or therapeutic treatment of *Streptococcus pyogenes* infection.

7. The pharmaceutical composition according to claim 6 wherein the antibody is a monoclonal antibody or polyclonal antibody.

8. The pharmaceutical composition according to claim 7 wherein the monoclonal antibody is a murine, rat or human antibody.

9. The pharmaceutical composition according to claim 6 wherein the antibody, or antigen-binding fragment thereof, is recombinant.

10. A pharmaceutical composition comprising (a) a pharmaceutically acceptable carrier or diluent, and (b) a chimeric polypeptide that comprises two or more fragments of the polypeptide consisting of the amino acid sequence set forth in Figure 2, wherein the two or more fragments each consist of at least 10 contiguous amino acids of Figure 2 and wherein the two or more fragments are linked to form a chimeric polypeptide, wherein the chimeric polypeptide is capable of raising antibodies that specifically bind to a protein consisting of the amino acid sequence set forth in Figure 2, and wherein the chimeric polypeptide is capable of eliciting an immune response against *Streptococcus pyogenes,* for use in the prophylactic or therapeutic treatment *of Streptococcus pyogenes* infection.

11. The pharmaceutical composition according to claim 10 further comprising a pharmaceutically acceptable adjuvant.

12. Use of the pharmaceutical composition selected from the group consisting of:
(a) a pharmaceutical composition comprising
(i) a polypeptide selected from (A) a polypeptide that comprises an amino acid sequence at least 95% identical to the amino acid sequence set forth in Figure 2; (B) a polypeptide that comprises the amino acid sequence set forth in Figure 2; and (C) a polypeptide comprising an immunogenic polypeptide fragment consisting of at least 10 contiguous amino acids of the amino acid sequence set forth in Figure 2,
wherein the polypeptide is capable of raising antibodies that specifically bind to a protein consisting of the amino acid sequence set forth in Figure 2, and wherein the polypeptide is capable of eliciting an immune response against *Streptococcus pyogenes,* and
(ii) a pharmaceutically acceptable carrier or diluent;
(b) a pharmaceutical composition comprising (i) an antibody, or antigen-binding fragments thereof, that specifically binds to a polypeptide consisting of the sequence set forth in Figure 2 wherein the antibody, or antigen-binding fragment thereof is capable of protecting a host from a *Streptococcus pyogenes* infection, and (ii) a pharmaceutically acceptable carrier or diluent; and
(c) a pharmaceutical composition comprising (i) a pharmaceutically acceptable carrier or diluent, and (ii) a chimeric polypeptide that comprises two or more fragments of the polypeptide consisting of the amino acid sequence set forth in Figure 2, wherein the two or more fragments each consist of at least 10 contiguous amino acids of Figure 2 and wherein the two or more fragments are linked to form a chimeric polypeptide, wherein the chimeric polypeptide is capable of raising antibodies that specifically bind to a protein consisting of the amino acid sequence set forth in Figure 2, and wherein the chimeric polypeptide is capable of eliciting an immune response against *Streptococcus pyogenes,*
in the manufacture of a medicament for the prophylactic or therapeutic treatment of a *Streptococcus pyogenes* infection.

13. A method for diagnosis of a *Streptococcus pyogenes* infection in a host susceptible to *S. pyogenes* infection comprising:
(a) incubating a biological sample previously obtained from the host with an antibody, or antigen-binding fragment thereof, reactive with a polypeptide consisting of the amino acid sequence set fort in Figure 2, to form a mixture; and
(b) detecting specifically bound antibody or bound antigen-binding fragment in the mixture which indicates the presence of *S. pyogenes* in the host.

14. A method for detecting an antibody that specifically binds to a *S. pyogenes* polypeptide, which polypeptide comprises the amino acid sequence set forth in Figure 2 in a biological sample containing or suspected of containing said antibody comprising:
a) incubating the biological sample with the polypeptide, to form a mixture; and
b) detecting specifically bound polypeptide in the mixture which indicates the presence of the antibody in the sample.

## Patentansprüche

1. Pharmazeutische Zusammensetzung, umfassend:
(a) ein Polypeptid ausgewählt aus (i) einem Polypeptid, das eine Aminosäuresequenz umfasst, die mit der in Figur 2 dargestellten Aminosäuresequenz zu mindestens 95 % identisch ist; und (ii) einem Polypeptid, umfassend ein immunogenes Polypeptidfragment, bestehend aus mindestens 10 zusammenhängenden Aminosäuren der in Figur 2 dargestellten Aminosäuresequenz,
wobei das Polypeptid in der Lage ist, Antikörper hervorzurufen, die spezifisch an ein Protein binden, das aus der in Figur 2 dargestellten Aminosäuresequenz besteht, und wobei das Polypeptid in der Lage ist, eine Immunantwort gegen Streptococcus pyogenes auszulösen, und
(b) ein/einen pharmazeutisch verträglichen/verträgliches Träger oder Verdünnungsmittel,
wobei die pharmazeutische Zusammensetzung zur Verwendung in der prophylaktischen oder therapeutischen Behandlung von Streptococcus pyogenes-Infektion dient.

2. Pharmazeutische Zusammensetzung gemäß Anspruch 1, wobei das Polypeptid die in Figur 2 dargestellte Aminosäuresequenz umfasst.

3. Pharmazeutische Zusammensetzung gemäß Anspruch 2, wobei dem Polypeptid der N-terminale Methioninrest fehlt.

4. Pharmazeutische Zusammensetzung gemäß mindestens einem der Ansprüche 1 bis 3, wobei die Zusammensetzung ferner ein pharmazeutisch verträgliches Adjuvans umfasst.

5. Pharmazeutische Zusammensetzung gemäß mindestens einem der Ansprüche 1 bis 3, wobei das Polypeptid rekombinant durch ein Verfahren hergestellt wird, das das Kultivieren einer Wirtszelle umfasst, transfiziert mit einem Vektor, der ein Polynukleotid umfasst, das das Polypeptid kodiert, wobei das Polynukleotid in funktionsfähiger Weise mit einer Expressions-Kontrollregion verknüpft ist.

6. Pharmazeutische Zusammensetzung, umfassend (a) einen Antiköper oder Antigen-bindendes Fragment davon, der/das spezifisch an ein Polypeptid bindet, das aus der in Figur 2 dargestellten Sequenz besteht, wobei der Antikörper oder das Antigen-bindende Fragment davon in der Lage ist, einen Wirt vor einer Streptococcus pyogenes-Infektion zu schützen, und (b) ein(en) pharmazeutisch verträglichen/verträgliches Träger oder Verdünnungsmittel, zur Verwendung in der prophylaktischen oder therapeutischen Behandlung von Streptococcus pyogenes-Infektion.

7. Pharmazeutische Zusammensetzung gemäß Anspruch 6, wobei der Antikörper ein monoklonaler Antikörper oder polyklonaler Antikörper ist.

8. Pharmazeutische Zusammensetzung gemäß Anspruch 7, wobei der monoklonale Antikörper ein muriner, Ratten- oder humaner Antikörper ist.

9. Pharmazeutische Zusammensetzung gemäß Anspruch 6, wobei der Antikörper oder das Antigen-bindende Fragment davon rekombinant ist.

10. Pharmazeutische Zusammensetzung, umfassend (a) ein(en) pharmazeutisch verträglichen/verträgliches Träger oder Verdünnungsmittel und (b) ein chimäres Polypeptid, das zwei oder mehr Fragmente des Polypeptids umfasst, das aus der in Figur 2 dargestellten Aminosäuresequenz besteht, wobei die zwei oder mehr Fragmente jeweils aus mindestens 10 zusammenhängenden Aminosäuren von Figur 2 bestehen und wobei die zwei oder mehr Fragmente verknüpft sind, um ein chimäres Polypeptid zu bilden, wobei das chimäre Polypeptid in der Lage ist, Antikörper hervorzurufen, die spezifisch an ein Protein binden, das aus der in Figur 2 dargestellten Aminosäuresequenz besteht, und wobei das chimäre Polypeptid in der Lage ist, eine Immunantwort gegen Streptococcus pyogenes auszulösen, zur Verwendung in der prophylaktischen oder therapeutischen Behandlung von Streptococcus pyogenes-Infektion.

11. Pharmazeutische Zusammensetzung gemäß Anspruch 10, die ferner ein pharmazeutisch verträgliches Adjuvans umfasst.

12. Verwendung der pharmazeutischen Zusammensetzung ausgewählt aus der Gruppe bestehend aus:
(a) einer pharmazeutischen Zusammensetzung, umfassend
(i) ein Polypeptid, ausgewählt aus (A) einem Polypeptid, das eine Aminosäuresequenz umfasst, die mit der in Figur 2 dargestellten Aminosäuresequenz zu mindestens 95 % identisch ist; (B) einem Polypeptid, das die in Figur 2 dargestellte Aminosäuresequenz umfasst; und (C) einem Polypeptid, umfassend ein immunogenes Polypeptidfragment, bestehend aus mindestens 10 zusammenhängenden Aminosäuren der in Figur 2 dargestellten Aminosäuresequenz,
wobei das Polypeptid in der Lage ist, Antikörper hervorzurufen, die spezifisch an ein Protein binden, das aus der in Figur 2 dargestellten Aminosäuresequenz besteht, und wobei das Polypeptid in der Lage ist, eine Immunantwort gegen Streptococcus pyogenes auszulösen, und
(ii) ein(en) pharmazeutisch verträglichen/verträgliches Träger oder Verdünnungsmittel;
(b) einer pharmazeutischen Zusammensetzung, umfassend
(i) einen Antikörper oder Antigen-bindendes Fragment davon, der/das spezifisch an ein Polypeptid bindet, das aus der in Figur 2 dargestellten Sequenz besteht, wobei der Antikörper oder das Antigen-bindende Fragment davon in der Lage ist, einen Wirt vor einer Streptococcus pyogenes-Infektion zu schützen, und (ii) ein(en) pharmazeutisch verträglichen/verträgliches Träger oder Verdünnungsmittel; und
(c) einer pharmazeutischen Zusammensetzung, umfassend
(i) ein(en) pharmazeutisch verträglichen/verträgliches Träger oder Verdünnungsmittel, und (ii) ein chimäres Polypeptid, das zwei oder mehr Fragmente des Polypeptids umfasst, das aus der in Figur 2 dargestellten Aminosäuresequenz besteht, wobei die zwei oder mehr Fragmente jeweils aus mindestens 10 zusammenhängenden Aminosäuren von Figur 2 bestehen und wobei die zwei oder mehr Fragmente verknüpft sind, um ein chimäres Polypeptid zu bilden, wobei das chimäre Polypeptid in der Lage ist, Antikörper hervorzurufen, die spezifisch an ein Protein binden, das aus der in Figur 2 dargestellten Aminosäuresequenz besteht, und wobei das chimäre Polypeptid in der Lage ist, eine Immunantwort gegen Streptococcus pyrogenes auszulösen,
in der Herstellung eines Medikaments zur prophylaktischen oder therapeutischen Behandlung einer Streptococcus pyogenes-Infektion.

13. Verfahren zur Diagnose einer Streptococcus pyogenes-Infektion in einem Wirt, der für S. pyogenes-Infektion anfällig ist, umfassend:
(a) Inkubieren einer vorher vom Wirt erhaltenen biologischen Probe mit einem Antikörper oder Antigen-bindenden Fragment davon, der/das mit einem Polypeptid reaktiv ist, das aus der in Figur 2 dargestellten Aminosäuresequenz besteht, um eine Mischung zu bilden; und
(b) Detektieren von spezifisch gebundenem Antikörper oder gebundenem Antigen-bindenden Fragment in der Mischung, was die Gegenwart von S. pyogenes im Wirt anzeigt.

14. Verfahren zum Detektieren eines Antikörpers, der spezifisch an ein S. pyogenes-Polypeptid bindet, wobei das Polypeptid die in Figur 2 dargestellte Aminosäuresequenz umfasst, in einer biologischen Probe, die den Antikörper enthält oder im Verdacht steht, ihn zu enthalten, umfassend:
(a) Inkubieren der biologischen Probe mit dem Polypeptid, um eine Mischung zu bilden; und
(b) Detektieren von spezifisch gebundenem Polypeptid in der Mischung, was die Gegenwart des Antikörpers in der Probe anzeigt.

## Revendications

1. Composition pharmaceutique comprenant :
(a) un polypeptide choisi parmi (i) un polypeptide qui comprend une séquence d'acides aminés identique à au moins 95 % à la séquence d'acides aminés présentée sur la figure 2 ; et (ii) un polypeptide comprenant un fragment de polypeptide immunogène consistant en au moins 10 acides aminés contigus de la séquence d'acides aminés présentée sur la figure 2,
dans laquelle le polypeptide peut élever des anticorps qui se lient spécifiquement à une protéine consistant en la séquence d'acides aminés présentée sur la figure 2, et dans laquelle le polypeptide peut provoquer une réponse immunitaire contre *Streptococcus pyogenes,* et
(b) un véhicule ou un diluant pharmaceutiquement acceptable,
dans laquelle la composition pharmaceutique est destinée à une utilisation dans le traitement prophylactique ou thérapeutique d'une infection par *Streptococcus pyogenes.*

2. Composition pharmaceutique selon la revendication 1, dans laquelle le polypeptide comprend la séquence d'acides aminés présentée sur la figure 2.

3. Composition pharmaceutique selon la revendication 2, dans laquelle le polypeptide ne comprend pas le résidu N-terminal de méthionine.

4. Composition pharmaceutique selon l'une quelconque des revendications 1 à 3, dans laquelle la composition comprend en outre un adjuvant pharmaceutiquement acceptable.

5. Composition pharmaceutique selon l'une quelconque des revendications 1 à 3, dans laquelle le polypeptide est produit par recombinaison par un procédé comprenant la culture d'une cellule hôte transfectée avec un vecteur qui comprend un polynucléotide codant pour le polypeptide, dans laquelle le polynucléotide est lié de façon opérationnelle à une région de contrôle d'expression.

6. Composition pharmaceutique comprenant (a) un anticorps, ou un fragment de liaison à un antigène de celui-ci, qui se lie spécifiquement à un polypeptide consistant en la séquence présentée sur la figure 2, dans laquelle l'anticorps, ou le fragment de liaison à un antigène de celui-ci, peut protéger un hôte contre une infection par *Streptococcus pyogenes,* et (b) un véhicule ou un diluant pharmaceutiquement acceptable, destinée à une utilisation dans le traitement prophylactique ou thérapeutique d'une infection par *Streptococcus pyogenes.*

7. Composition pharmaceutique selon la revendication 6, dans laquelle l'anticorps est un anticorps monoclonal ou un anticorps polyclonal.

8. Composition pharmaceutique selon la revendication 7, dans laquelle l'anticorps monoclonal est un anticorps murin, de rat ou humain.

9. Composition pharmaceutique selon la revendication 6, dans laquelle l'anticorps, ou un fragment de liaison à un antigène, est recombinant.

10. Composition pharmaceutique comprenant (a) un véhicule ou un diluant pharmaceutiquement acceptable, et (b) un polypeptide chimère qui comprend deux fragments ou plus du polypeptide consistant en la séquence d'acides aminés présentée sur la figure 2, dans laquelle les deux fragments ou plus consistent chacun en moins 10 acides aminés contigus de la figure 2 et dans laquelle les deux fragments ou plus sont liés pour former un polypeptide chimère, dans laquelle le polypeptide chimère peut élever des anticorps qui se lient spécifiquement à une protéine consistant en la séquence d'acides aminés présentée sur la figure 2, et dans laquelle le polypeptide chimère peut provoquer une réponse immunitaire contre *Streptococcus pyogenes,* destinée à une utilisation dans le traitement prophylactique ou thérapeutique d'une infection par *Streptococcus pyogenes.*

11. Composition pharmaceutique selon la revendication 10, comprenant en outre un adjuvant pharmaceutiquement acceptable.

12. Utilisation de la composition pharmaceutique choisie dans le groupe consistant en :
(a) une composition pharmaceutique comprenant
(i) un polypeptide choisi parmi (A) un polypeptide qui comprend une séquence d'acides aminés identique à au moins 95 % à la séquence d'acides aminés présentée sur la figure 2 ; (B) un polypeptide qui comprend la séquence d'acides aminés présentée sur la figure 2 ; et (C) un polypeptide comprenant un fragment de polypeptide immunogène consistant en au moins 10 acides aminés contigus de la séquence d'acides aminés présentée sur la figure 2,
dans laquelle le polypeptide peut élever des anticorps qui se lient spécifiquement à une protéine consistant en la séquence d'acides aminés présentée sur la figure 2, et dans laquelle le polypeptide peut provoquer une réponse immunitaire contre *Streptococcus pyogenes,* et
(ii) un véhicule ou un diluant pharmaceutiquement acceptable ;
(b) une composition pharmaceutique comprenant (i) un anticorps, ou un fragment de liaison à un antigène de celui-ci, qui se lie spécifiquement à un polypeptide consistant en la séquence présentée sur la figure 2, dans laquelle l'anticorps, ou le fragment de liaison à un antigène de celui-ci, peut protéger un hôte contre une infection par *Streptococcus pyogenes,* et (ii) un véhicule ou un diluant pharmaceutiquement acceptable ; et
(c) une composition pharmaceutique comprenant (i) un véhicule ou un diluant pharmaceutiquement acceptable, et (ii) un polypeptide chimère qui comprend deux fragments ou plus du polypeptide consistant en la séquence d'acides aminés présentée sur la figure 2, dans laquelle les deux fragments ou plus consistent chacun en au moins 10 acides aminés contigus de la figure 2 et dans laquelle les deux fragments ou plus sont liés pour former un polypeptide chimère, dans laquelle le polypeptide chimère peut élever des anticorps qui se lient spécifiquement à une protéine consistant en la séquence d'acides aminés présentée sur la figure 2, et dans laquelle le polypeptide chimère peut provoquer une réponse immunitaire contre *Streptococcus pyogenes,*
dans la fabrication d'un médicament pour le traitement prophylactique ou thérapeutique d'une infection par *Streptococcus pyogenes.*

13. Procédé de diagnostic d'une infection par *Streptococcus pyogenes* chez un hôte sensible à une infection par *S. pyogenes* comprenant :
(a) l'incubation d'un échantillon biologique obtenu au préalable auprès d'un hôte avec un anticorps, un fragment de liaison à un antigène de celui-ci, réactif avec un polypeptide consistant en la séquence d'acides aminés présentée sur la figure 2, pour former un mélange ; et
(b) la détection de l'anticorps lié spécifiquement ou du fragment de liaison à un antigène lié dans le mélange, ce qui indique la présence de *S. pyogenes* chez l'hôte.

14. Procédé de détection d'un anticorps qui se lie spécifiquement à un polypeptide de *S. pyogenes,* lequel polypeptide comprend la séquence d'acides aminés présentée sur la figure 2, dans un échantillon biologique contenant ou susceptible de contenir ledit anticorps, comprenant :
a) l'incubation de l'échantillon biologique avec le polypeptide pour former un mélange ; et
b) la détection du polypeptide lié spécifiquement dans le mélange, ce qui indique la présence de l'anticorps dans l'échantillon.
